(19) **Europäisches Patentamt / European Patent Office / Office européen des brevets**

(11) **EP 4 663 180 A1**

(12) # EUROPEAN PATENT APPLICATION

(43) Date of publication:
**17.12.2025 Bulletin 2025/51**

(21) Application number: **24382624.5**

(22) Date of filing: **10.06.2024**

(51) International Patent Classification (IPC):
$A61K\ 8/9789^{(2017.01)}$    $A61K\ 36/48^{(2006.01)}$
$A61P\ 17/00^{(2006.01)}$    $A61P\ 17/06^{(2006.01)}$
$A61P\ 17/10^{(2006.01)}$    $A61Q\ 17/04^{(2006.01)}$

(52) Cooperative Patent Classification (CPC):
(C-Sets available)
**A61Q 17/04; A61K 8/9789; A61K 36/48;**
**A61P 17/00; A61P 17/06; A61P 17/10;**
A61K 2800/5922      (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Industrial Farmaceutica Cantabria, S.A.**
**39690 La Concha de Villaescusa (ES)**

(72) Inventors:
• **Rodríguez Navarro, Susana**
**39690 La Concha de Villaescusa (ES)**

• **López Sánchez, Ana**
**39690 La Concha de Villaescusa (ES)**
• **Cáceres Estévez, Irene**
**39690 La Concha de Villaescusa (ES)**
• **González Rodríguez, Salvador**
**39690 La Concha de Villaescusa (ES)**
• **Haya Rodríguez, Luisa**
**39690 La Concha de Villaescusa (ES)**
• **Nieto Botella, Luis**
**39690 La Concha de Villaescusa (ES)**

(74) Representative: **Clarke, Modet y Cía., S.L.**
**C/ Suero de Quiñones 34-36**
**28002 Madrid (ES)**

(54) **SYNERGIC COMBINATION OF GREEN ASPALATHUS LINEARIS AND RED ASPALATHUS LINEARIS EXTRACTS FOR USE IN TREATING A SUN RADIATION-INDUCED DISEASE**

(57) The present invention is a synergic combination of green *Aspalathus linearis* (RBG) and red *Aspalathus linearis* (RBR) extracts for use in the prophylaxis or treatment of a sun radiation-induced disease or disorder in a subject, its use in a cosmetic and non-therapeutical treatment of skin, mucous membranes, scalp and/or hair, and the pharmaceutical, nutraceutical and cosmetic compositions containing said combination.

Figure 2

(52) Cooperative Patent Classification (CPC): (Cont.)

C-Sets
**A61K 36/48, A61K 2300/00**

**Description**

**Field of the invention**

The sector of the present invention is the pharmacological industry, in particular the preparation of a pharmaceutical, cosmetic or nutraceutical composition for photoprotection.

**Background of the invention**

Excessive exposure to solar radiation has negative consequences for skin health, including DNA damage, inflammation, immunosuppression, carcinogenesis, and photoaging. This can be due to direct damage to the DNA or also indirectly mediated by the generation of Reactive Oxygen Species (ROS). Since continuous radiation exposure overwhelms the skin's endogenous repair and antioxidant mechanisms, the art is in the unceasing search for innovative approaches to prevent DNA damage, protecting and enhancing the skin's endogenous repair and antioxidant systems, paying special attention to natural extracts containing polyphenols, carotenoids, alkaloids, repair enzymes, etc., as skin protective agents.

In this context, *Aspalathus linearis* (Brum.f.) Dahlg., is an endemic bush from Cederberg region in South Africa, popularly known as rooibos, with important bioactive reported antioxidant properties, among others (Abdul NS, Marnewick JL. What has been the focus of Rooibos health research? A bibliometric overview. J Herb Med., 2023;37:100615).

Its remarkable antioxidant activity derives from a composition rich in flavonoids, where C and O-glycosides are key. Of special interest are three dihydrochalcones: Aspalathin and linearthin, specific from the species (*Aspalathus linearis*), and nothofagin - restricted to few plant species apart from rooibos. Rooibos also contains certain isomeric flavones such as orientin, isorientin and isovitexin, as well as other flavonoids such as luteolin in lower proportions, and the flavonols quercetin, isoquercetin and rutin. Relevant is that these compounds are not usually standardized in the different forms of rooibos extracts/teas.

For the traditional use as tea, leaves and shoots from the bush are first subjected to a process in which the plant tissues get dry and oxidize, known as "fermentation". In this process, many of the mentioned components present in the fresh plant tissue are transformed.

Assuming the variability ascribable to differences during the farming and fermentation processes, it is generally accepted that the amount of aspalathin and nothofagin are high in the fresh tissue, but importantly decrease during fermentation, being very scarce in fermented rooibos. Consequently, two main types of rooibos are identified. On one hand, the traditional type subjected to the fermentation process, known as "fermented" or "red" rooibos (hereinafter RBR); and on the other, the "non-fermented" or "green" rooibos (hereinafter RBG).

Due to the strong antioxidant character of aspalathin, nothofagin and linearthin, it is generally accepted that RBG extracts possess a much higher antioxidant capacity compared to RBR. In fact, RBG showed 28% higher total radical trapping antioxidant parameter (TRAP) in *in vitro* measurements than RBR. Since these compounds have low water solubility, the main part of studies associating important biological activities to rooibos used organic extracts from RBG, correlating biological benefits with the antioxidant levels.

Akinfenwa reported certain biological activity of organic extractions of RBG and its main cytoprotectant compounds under UVB irradiation, relating the biological effect with the antioxidant capacity of the previously mentioned characteristic dihydrochalcones (Akinfenwa AO. Protective Effects of Linearthin and Other Chalcone Derivatives from Aspalathus linearis (Rooibos) against UVB Induced Oxidative Stress and Toxicity in Human Skin Cells. Plants Basel Switz., 2021; 10(9):1936).

However, even when it is supposed to possess much lower antioxidant activity compared to RBG and just a little amount of the typical compounds of interest, RBR water extractions similar to the traditional tea use and with no reported photoprotective effects, have a strong safety profile supported by a widespread consumption, which can explain why most clinical studies with rooibos up to date have used this extract.

WO 2010000579 A specifically discloses the use of rooibos extract for to improve UV protection or reduce skin wrinkles or related skin inflammation, in particular in the form of oral administration. The extract is a water or ethanol/water extraction of typically fermented leaves and step pieces, indicating that these results were obtained with RBR. No reference to RBG is made and no results reported therefrom, much less a combination of the two biological materials.

The detailed composition of RBG and RBR has been disclosed and compared by Bednarska (Bednarska K. and Fecka I., "Aspalathin and Other Rooibos Flavonoids Trapped α-Dicarbonyls and Inhibited Formation of Advanced Glycation End Products In Vitro", 2022, Int. J. Mol. Sci., 23, 14738). The document discloses the antiglycation activity of the main components of each and discusses the effect of the infusion and of the hydroethanolic extract of RBG and RBR in related chronic metabolic diseases like diabetes. It does not disclose that both extracts could be combined for a better therapeutical result, though.

The problem of the art can be considered as how to find natural compositions containing Rooibos with enhanced photoprotective properties. The solution proposed by the present invention is a combination of green Rooibos (RBG) and red oxidized Rooibos (RBR) extracts for a synergic effect.

**Description of the invention**

The present invention is the combination of a green *Aspalathus linearis* (RBG) extract and a red *Aspalathus linearis* (RBR) extract, for use in the prophylaxis or treatment of a sun radiation-induced disease or disorder in a subject.

Another aspect of the invention is the use of a combination of a green *Aspalathus linearis* (RBG) extract and a red *Aspalathus linearis* (RBR) extract in the preparation of a medicament for the prophylaxis or treatment of a sun radiation-induced disease or disorder in a subject. And still a further aspect is a method of treating or preventing a sun radiation-induced disease or disorder in a subject comprising administering to the subject in need thereof a therapeutically effective amount of a combination of a green *Aspalathus linearis* (RBG) extract and a red *Aspalathus linearis* (RBR) extract.

In a preferred aspect, said prophylaxis or treatment is photoprotection of skin cells, mucous membranes, preferably eye mucous membrane, scalp and/or hair.

In another preferred aspect, said disease or disorder is photoaging, cell damage, thermal stress, oxidative stress, osmotic shock, inflammation, exposure to pollutants, lack of skin nourishment and lack of hydration, a skin pigmentation disorder, vitiligo, healing and/or re-epithelialization of wounds, reactive or dry skin, psoriasis, or acne.

Another preferred aspect is that said disease or disorder courses with the generation of Reactive Oxygen Species (ROS).

Suitable extracts of both RBG and RBR *Aspalathus linearis* are prepared by any means known in the art, e.g., by steam extraction, solvent extraction, distillation, pressing or grinding, or a combination thereof. In an aspect of the invention, the extracts can be organic extracts. In a preferred aspect, the extracts of the plant are aqueous extracts or hydroalcoholic extracts, even more preferably water extracts. Suitable extracts of *Aspalathus linearis* for the purpose of the present invention are also commercially available extracts, for example Rooibos tea of RBR.

In a preferred aspect, the extracts used in the present invention are from 0.01%, to 100% water soluble, more preferably at least 95% water soluble.

In the present application, the Green *Aspalathus linearis* is defined as the natural (non-fermented) *Aspalathus linearis* plant identified as RBG.

In the present application, the Red *Aspalathus linearis* is defined as the oxidized (fermented) *Aspalathus linearis* plant, identified as RBR. Typically, the RBR is obtained by harvesting the plants cutting their stems and leaves and putting them in a heap. The temperature inside the heap rapidly raises 40°C and causes the fermentation of the leaves. After about one day, the leaf and stem pieces are dried in the sun and sieved, ready for infusion. This process is progressively replaced by batch rotary fermentation of moistened leaves and stems, after which the sun-drying step is maintained.

The extracts combination of the present invention surprisingly shows a synergic effect in the prophylaxis or treatment of sun induced diseases, or protection in a cosmetic use.

In the present application, a synergic effect of the RBG and RBR extracts is defined as the cooperation of said extracts to produce a combined effect in the subject that is greater than the sum of the effect of each extract considered separately.

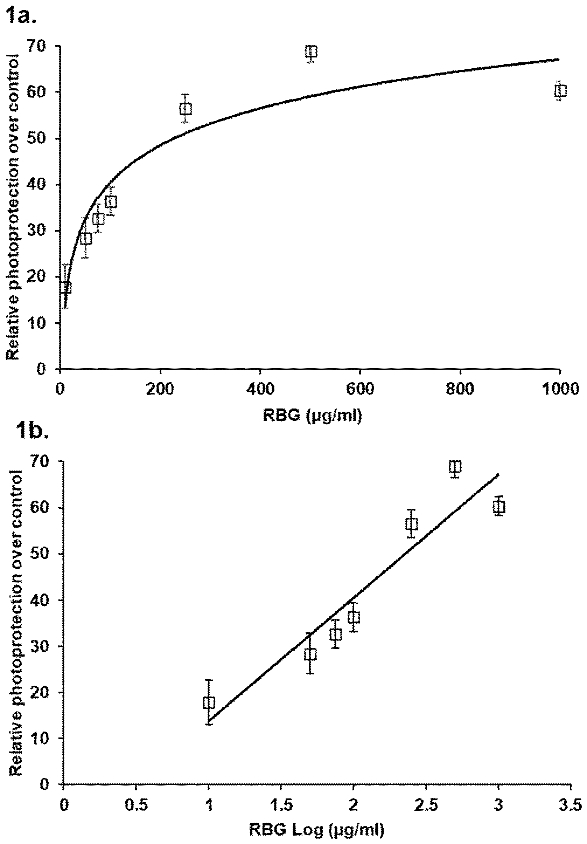

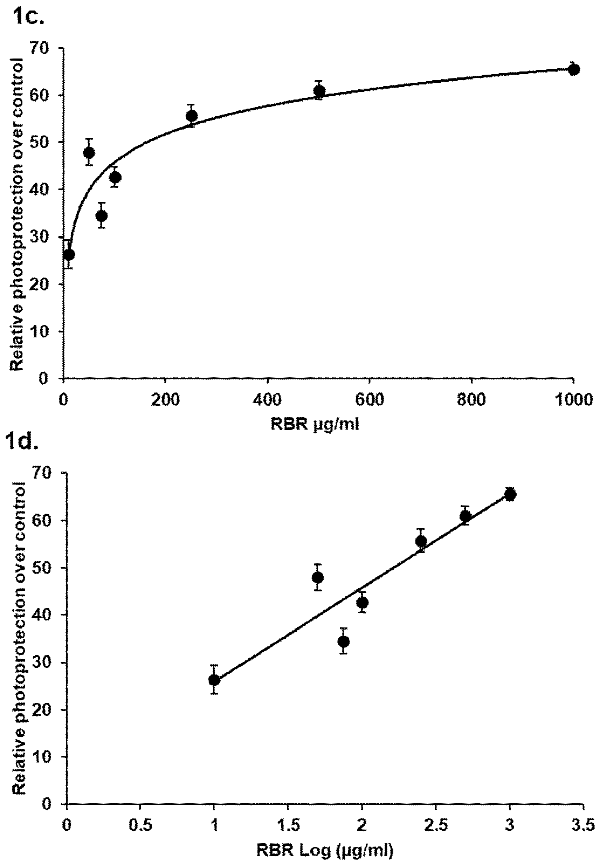

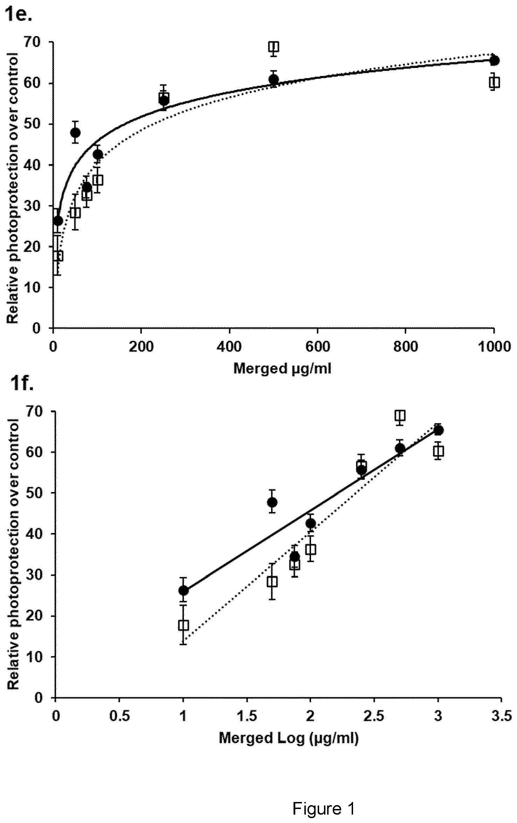

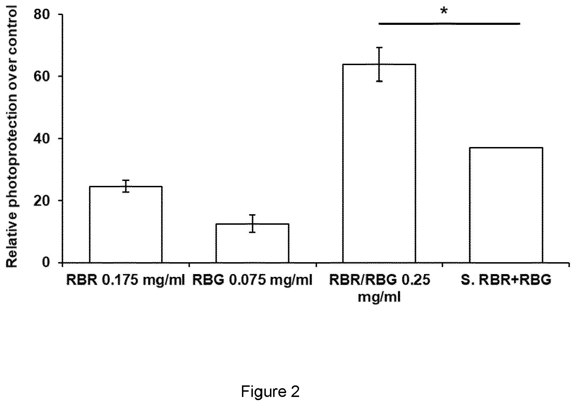

In the experimental setup of Example 2 below, the photoprotective effect of RBG resulted comparable with that reported by Akinfenwa et al. 2021 as referenced, validating the model and the use in the art of aqueous RBG extract for photoprotective purposes (Figures 1a and b). Interestingly, aqueous RBR showed a strong photoprotective effect (Figures 1c and d) of a magnitude even higher than RBG (Figure 1e and f), since lower concentrations showed higher photoprotection. The synergic effect of the combination RBR:RBG in photoprotection is demonstrated by the results of Example 3, showing higher levels than the simple sum "S. RBR+RBG" (Figure 2).

However, neither the photoprotective effect of RBR nor of the synergystic RBR:RBG can be assigned to the qualitative composition, as demonstrated in Examples 4 and 5. The quantitative analysis of the extracts and/or their antioxidant capacity alone do not explain the photoprotection activity exhibited in the treatment; rather, the observed induced photoprotection is plausibly emerging from the endogenous photoprotective mechanisms of the cells when enhanced by the extracts. It is also remarkable that the photoprotective effect observed in RBR does not correlate well with the antioxidant capacity of the extract. Considering that the combination RBR:RBG showed no synergic antioxidant capacity, altogether the reported data indicate that the synergic effect observed for photoprotection cannot be explained by an antioxidant effect either.

In an aspect of the invention, the relative content of RBR vs. RBG in the combination of the invention is from 99:1 to 1:99 wt%, preferably from 90:10 to 10:90 wt%, most preferably RBR:RBG of 70:30 wt%.

Another aspect of the invention is a pharmaceutical, nutraceutical or cosmetic composition comprising a combination of RBG and RBR extracts and at least one pharmaceutical or nutraceutical, or cosmetical acceptable excipient or adjuvant. Preferably, the composition is for topical or oral administration.

The composition for topical administration preferably contains the combination of the invention in an amount of 0.1 to 10 wt%, or 0.2 to 5 wt%, or 0.2 to 1 wt%, most preferably of 0.25 wt% or 0.5 wt% of the total weight of the composition.

The composition for oral administration preferably contains the combination of the invention in an amount of 1 to 90 wt%, or 10 to 50 wt%, or 20 to 30 wt%, most preferably of 25 wt% of the total weight of the composition.

The combination of the invention can be supplemented with other plant extracts also useful in photoprotection.

In this sense, one of the most successfully used additional ingredient with a similar Mode of Action (MOA) as observed for RBR:RBG is the extract of *Polypodium leucotomos* (EPL), which renders as a cosmetic and nutraceutical systemic photoprotectant, with a wide range of clinical properties.

Therefore, a preferred aspect is the combination of the RBR:RBG extracts of the invention with other plant extracts, preferably an aqueous extract of EPL. The photoprotective activity of EPL has been associated with the presence, among others, of phenolic acids such as 3,4-Dihydroxybenzoic, 4-Hydroxybenzoic, Vanillic Acid (3-methyl-4-hydroxybenzoic), Caffeic Acid (3,4-dihydroxycinnamic), 4-hydroxycinnamic, 4-hydroxycinnamoyl-quinic, Ferulic (3-methyl-4-hydroxy cinnamic) and different chlorogenic (Caffeoyl-quinic).

Another preferred aspect is an oral composition comprising the combination of the invention in the form of a food product, a drink, a food supplement, a nutraceutical, a pet food or a medicament, even more preferably containing an aqueous extract of EPL.

The combination of the present invention is to be used in an animal, more preferably a companion animal or livestock, and most preferably a human.

Another preferred aspect is the cosmetic non-therapeutical use of a composition comprising the combination of the invention in the treatment or care of skin, mucous membranes, scalp and/or hair.

A preferred aspect of the invention is a pharmaceutical, nutraceutical or cosmetic composition in which the RBR and RBG extracts are incorporated into a delivery system or pharmaceutically or cosmetically acceptable sustained release system selected from the group consisting of liposomes, mixed liposomes, oleosomes, millicapsules, microcapsules, nanocapsules, sponges, cyclodextrines, vesicles, micelles, mixed micelles of surfactants, surfactant-phospholipid mixed micelles, millispheres, microspheres, nanospheres, lipospheres, microemulsions, nanoemulsions, milliparticles, microparticles, nanoparticles and solid lipid nanoparticles and/or is adsorbed on a cosmetic or pharmaceutically acceptable solid organic polymer or solid support selected from the group consisting of starch and maltodextrin.

In another preferred aspect, the pharmaceutical, nutraceutical or cosmetic composition comes in a formulation selected from the group consisting of creams, multiple emulsions, anhydrous compositions, aqueous dispersions, oils, milks, balsams, foams, lotions, gels, cream gels, hydroalcoholic solutions, hydroglycolic solutions, liniments, sera, soaps, shampoos, conditioners, serums, ointments, mousses, pomades, powders, bars, pencils, sprays, capsules, soft gelatin capsules, tablets, coated tablets, granulated forms, chewing gum, solutions, suspensions, emulsions, syrups, polysaccharide films, jelly and gelatin.

In another preferred aspect, the pharmaceutical, nutraceutical or cosmetic composition is incorporated into a product selected from the group consisting of concealers, makeup foundations, make-up removal lotions, make-up removal milks, eye shadows, lipsticks, lip glosses, lip protectors and powders. In a further preferred aspect, the pharmaceutical or cosmetic composition is incorporated in a fabric, a nonwoven fabric or a medical device.

Still in another preferred aspect, the pharmaceutical or cosmetic composition comprises a cosmetically or pharmaceutically effective amount of at least one adjuvant selected from the group consisting of an Reactive Carbonyl Scavenger (RCS), Matrix metalloprotease (MMP) inhibiting agent, melanin synthesis stimulating or inhibiting agents, whitening or depigmenting agents, propigmenting agents, self-tanning agents, antiaging agents, inhibiting agents of NO-synthase, inhibiting agents of $5\alpha$ reductase, inhibitor agents of lysyl- and/or prolyl-hydroxylase, antioxidants, free radical scavengers and/or agents against atmospheric pollution, anti-glycation agents, antihistamine agents, antiemetic agents, antiviral agents, antiparasitic agents, emulsifiers, emollients, organic solvents, liquid propellants, skin conditioners, humectants, substances that retain moisture, alpha hydroxy acids, beta hydroxy acids, moisturizers, epidermal hydrolytic enzymes, vitamins, pigments or colorants, dyes, gelling polymers, thickeners, surfactants, softening agents, anti-wrinkle agents, agents able to reduce or treat the bags under the eyes, exfoliating agents, antimicrobial agents, antifungal agents, fungistatic agents, bactericidal agents, bacteriostatic agents, agents stimulating the synthesis of dermal or epidermal macromolecules and/or capable of inhibiting or preventing their degradation, agents stimulating the synthesis of collagen, agents stimulating the synthesis of elastin, agents stimulating the synthesis of decorin, agents stimulating the synthesis of laminin, agents stimulating the synthesis of defensins, agents stimulating the synthesis of chaperones, agents stimulating the synthesis of aquaporins, agents stimulating the synthesis of hyaluronic acid, agents stimulating the synthesis of fibronectin, agents stimulating the synthesis of sirtuins, agents stimulating the synthesis of lipids and components of the stratum corneum, agents that inhibit collagen degradation, agents that inhibit elastin degradation, agents that inhibit serine proteases such as leukocyte elastase or cathepsin G, agents stimulating fibroblast proliferation, agents stimulating keratinocyte proliferation, agents stimulating adipocyte proliferation, agents stimulating melanocyte proliferation, agents stimulating keratinocyte differentiation, agents stimulating adipocyte differentiation, agents that inhibit acetylcholinesterase, skin relaxant agents, agents stimulating the synthesis of glycosaminoglycans, antihyperkeratosis agents, comedolytic agents, antipsoriasis agents, DNA repairing agents, DNA protecting agents, stabilizers, anti-itching agents, agents for the treatment and/or care of sensitive skin, firming agents, anti-stretch mark agents, binding agents, agents regulating sebum production, lipolytic agents or agents stimulating lipolysis, anti-cellulite agents, antiperspirant agents, agents stimulating healing, coadjuvant healing agents, agents stimulating reepithelialization, coadjuvant reepithelialization agents, cytokine growth factors, calming agents, anti-inflammatory agents, anesthetic agents, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, preservatives, perfumes, chelating agents, other plant extracts, essential oils, marine extracts, agents obtained from a biofermentation process, mineral salts, cell extracts and sunscreens, other organic or mineral photoprotection agents active against ultraviolet A and/or B rays, or a mixture thereof.

## Brief description of the figures

**Figure 1.** Shows the photoprotective effect of aqueous extracts of RBG and RBR according to Example 2. For all samples $n \geq 3$ independent wells of cells. Error bars represent standard error of mean (SEM). Y axis in all the graphs represents the photoprotective effect shown by the fibroblasts. X axis represents the concentration of the extracts used in the treatment, linearly expressed in graphs a, c and e, and in a logarithmic scale in b (y=26.695x-12.916, $R^2$=0.8865), d (y=19.834x+6.1587, $R^2$=0.8693) and f. Accordingly, data trendlines were drawn. **a.** and **b.** Photoprotection induced by RBG treatments ($\square$); **c.** and **d.** Photoprotection induced by RGR treatments (•); **e.** Overlapping of the previous graphs a. (RBG -$\square$-) and c. (RBR -•-); **f.** Overlapping of b. (RBG -$\square$-) and d. (RBR -•-).

**Figure 2.** Shows the synergic photoprotective effect of a combination of RBR and RBG. The bar graph represents the average contribution of each treatment in photoprotection. Error bars represent SEM. Y axis represents the photoprotective effect showed by the fibroblasts and analyzed as described in Example 2. Asterisk represents p-value $\leq 0.001$ (Student's t-Test). n = 16 independent wells of cells/group.

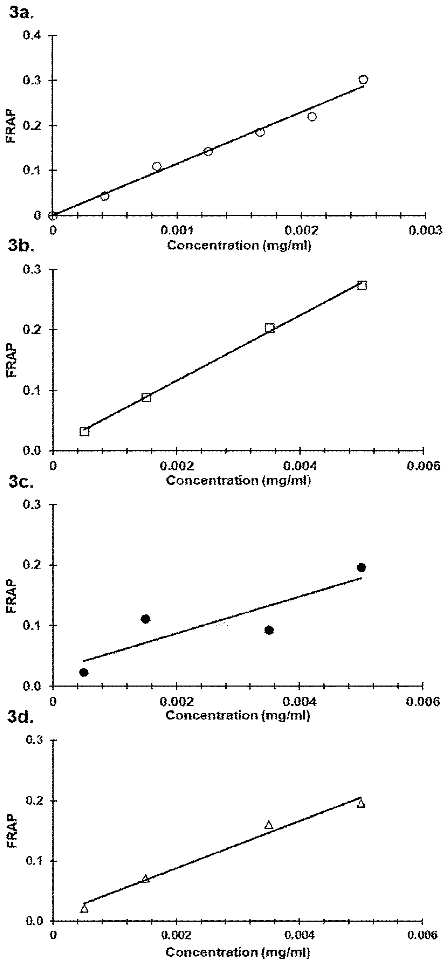

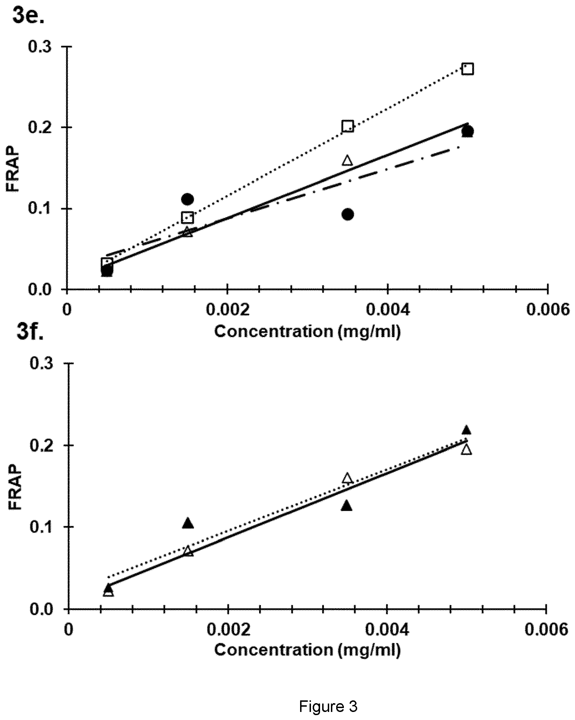

**Figure 3.** Antioxidant capacity of RBR and RBG analyzed by ferric ion reducing antioxidant power (FRAP). FRAP assay was performed to determine the antioxidant capacity of each extract, RBR, RBG, and their combination in a 70:30 wt% proportion respectively. The lineal graph representations show the activity of each sample depending on the concentration. Y axes represent the absorbance obtained by the plate reader at 593nm minus the blank. X axes represented the concentrations (mg/ml) at which the extracts were tested.
**a.** Lineal FRAP representation of Trolox (o) as an antioxidant reference (positive control) (y=114.48x+0.0006, $R^2$=0.9873). **b.** Lineal FRAP representation of the antioxidant activity of RBG ($\square$). **c.** Lineal FRAP representation of the antioxidant activity of RBR (•). **d.** Lineal FRAP representation of the antioxidant activity of RBR-RBG at 70:30 wt% proportion ($\triangle$). **e.** Lineal FRAP comparative representation of the antioxidant activity of RBR (•), RBG ($\square$) and RBR-RBG ($\triangle$) (70:30). **f.** Lineal FRAP comparative representation of the antioxidant activity of the theoretical RBR-RBG (A, calculated assuming linearity from the values obtained in the individual extract of RBR and RBG, and counting on its 70:30 proportion in the combination), against the experimental measures of RBR-RBG (70:30, $\triangle$ from "e").

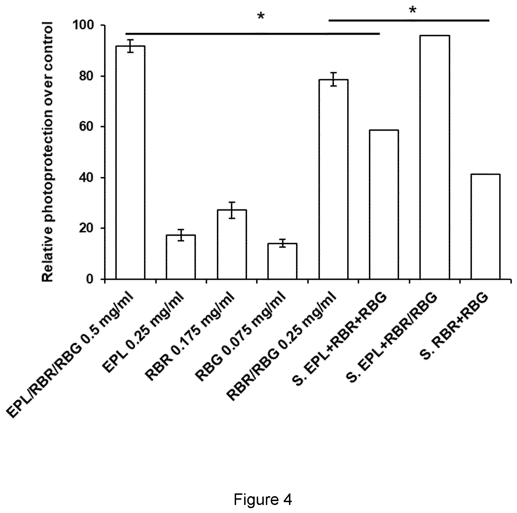

**Figure 4.** Shows the photoprotective effect of RBR, RBG, EPL and a combination of RBG, RBR and EPL. The assay is performed in a normal human dermal fibroblast culture as described in Example 2. For all samples $n \geq 11$ independent wells of cells. Error bars represent SEM. Y axis in all the graphs represents the induced-photoprotective effect showed by the fibroblasts. Asterisks represent p-value $\leq 0.001$ (Student's t-Test).

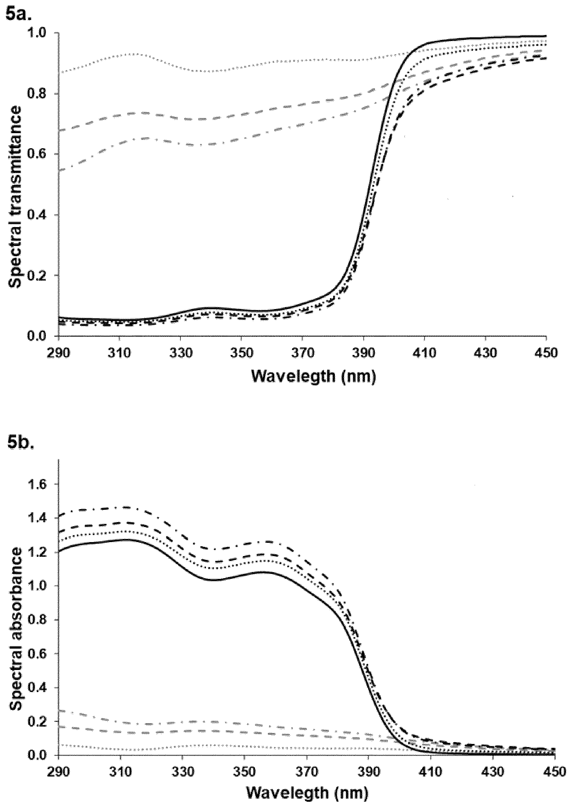

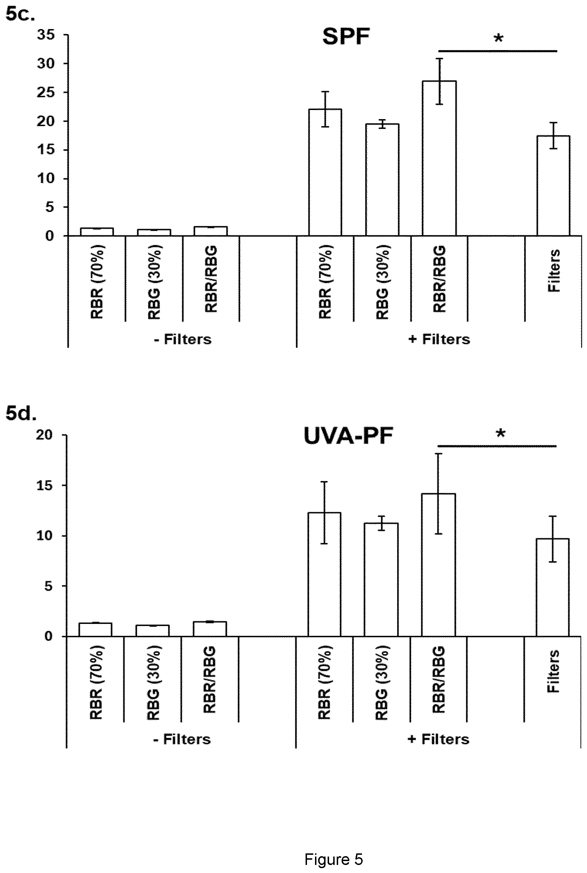

**Figure 5.** Shows the UV filter and booster effect of RBR, RBG, and its combination in topical sunscreen formulations. The sunscreen effect was analyzed by including 2.5% of the different extracts in galenic combinations containing +/- organic filters (+/- filters). **a.** Spectral transmittance in the UV and HEV (high-energy visible light) spectral regions (290-450 nm). **b.** Spectral absorbance in UV and HEV spectral regions (290-450 nm). For a. and b., RBR-70% (- - ) grey, RBG-30% (····) grey, RBR-70%+RBR-30% (-·-·) grey, RBR-70%+filters ( - - ) black, RBG-30%+filters (····) black, RBR-70%+RBR-30%+filters (-·-·) black, Filters (---) black. **c.** In vitro Sun Protection Factor (SPF). **d.** UVA protection factor (UVA-PF). C and d were estimated following the same protocol as ISO 24443:2012 for in vitro UVA-PF determination. For all samples, data points are representative of 4 independent replicates n=4, each one average of 3 readings. Error bars represent +/- standard deviation (SD). Asterisks represent p value $\leq 0.01$ (Student's t-Test).

## Examples

## Example 1. Preparation of the extracts

**Extracts origin:** *Polypodium leucotomos* solid extract used in the experiments described below was provided by Industrial Farmaceutica Cantabria, S.A., Madrid, Spain. RBR and RBG solid extracts used were supplied by Rooibos Ltd.

Sample preparation for Examples 2, 3, 5, and Example 4 at the antioxidant capacity

Solid extracts were weighed, suspended in HzOmq, and stirred for 30 minutes. Samples went through a 0,45$\mu$m PVDF membrane filter, and diluted in H$_2$Omq, proportional to the desired concentrations.

Sample preparation for Example 4 at the chemical composition of the extracts

Accurately weigh approximately 175$\pm$10 mg of RBR and transfer to a 100 ml topaz volumetric flask. Add 0.5 ml of 10% ascorbic acid solution, make up to volume with dissolution medium and sonicate for 10 minutes. Finally, the sample solution was filtered through a 0.45 $\mu$m PVDF filter. Accurately weight approximately 150$\pm$10 mg of RBG and transfer to a 200 ml topaz volumetric flask. Add 1.0 ml of 10% ascorbic acid solution, make up to volume with dissolution medium and sonicate for 10 minutes. Finally, the sample solution was filtered through a 0.45 $\mu$m PVDF filter.

Sample preparation for Example 6

- Sample RBR (70%): Oil/water emulsion was formulated containing a mixture of Neo PCL self-emulsifying base (Cetearyl Ethylhexanoate, Ceresin, Lanolin, Sorbitan Sesquioleate, Stearyl Heptanoate, Hydrogenated Castor Oil, Stearyl Caprylate) and propylene glycol in water as the base formula. *Aspalathus linearis* extract (RBR Extract) was added to the propylene glycol-water phase prior to mixing with the Neo PCL. The weight ratio of the ingredients in the final formula was 20% w/w Neo PCL, 5% w/w Propylene glycol, 1.75% w/w *Aspalathus linearis* extract (RBR Extract), and 73.25% w/w water. The galenic compositions were formulated at 60 °C.
- Sample RBG (30%): %): Oil/water emulsion was formulated containing a mixture of Neo PCL self-emulsifying base (Cetearyl Ethylhexanoate, Ceresin, Lanolin, Sorbitan Sesquioleate, Stearyl Heptanoate, Hydrogenated Castor Oil, Stearyl Caprylate - Acofarma, Barcelona, Spain) and propylene glycol in water as the base formula. *Aspalathus linearis* extract (RBG Extract) was added to the propylene glycol-water phase prior to mixing with Neo PCL. The weight ratio of the ingredients in the final formula was 20% w/w Neo PCL, 5% w/w Propylene glycol, 0.75% w/w *Aspalathus linearis* extract (RBG Extract) and 74.25% w/w water. The galenic compositions were formulated at 60 °C.
- Sample RBR/RBG (RBR:RBG 70:30%): Oil/water emulsion was formulated containing a mixture of NEO PCL (Acofarma, Barcelona, Spain) self-emulsifying base and propylene glycol in water as the base formula. *Aspalathus linearis* extract (RBR Extract) and *Aspalathus linearis* extract (RBG Extract) were added to the propylene glycol-water phase prior to mixing with Neo PCL. The weight ratio of the ingredients in the final formula was 20% w/w Neo PCL, 5% w/w Propylene glycol, 1.75% w/w *Aspalathus linearis* extract (RBG Extract), 0.75% w/w *Aspalathus linearis* extract (RBG Extract) and 72.5% w/w water. The galenic compositions were formulated at 60 °C.
- Sample Filters: Oil/water emulsion was formulated containing a mixture of Neo PCL self-emulsifying base (Acofarma, Barcelona, Spain) and propylene glycol in water as the base formula. Ethylhexyl Methoxycinnamate and Butyl Methoxydibenzoylmethane were added to the propylene glycol-water phase prior to mixing with Neo PCL. The weight ratio of the ingredients in the final formula was 20% w/w NEO PCL, 5% w/w Propylene glycol, 4% Ethylhexyl Methoxycinnamate and 2.5% Butyl Methoxydibenzoylmethane and 68.5% w/w water. The galenic compositions were formulated at 60 °C.
- Sample RBG (30%) + Filters: Oil/water emulsion was formulated containing a mixture of NEO PCL self-emulsifying base (Acofarma, Barcelona, Spain) and propylene glycol in water as the base formula. Ethylhexyl Methoxycinnamate, Butyl Methoxydibenzoylmethane and *Aspalathus linearis* extract (RBR Extract) were added to the propylene glycol-water phase prior to mixing with Neo PCL. The weight ratio of the ingredients in the final formula were 20% w/w Neo PCL, 5% w/w Propylene glycol, 4% Ethylhexyl Methoxycinnamate, 2.5% w/w Butyl Methoxydibenzoylmethane, 0.75% w/w *Aspalathus linearis* extract (RBG Extract), and 67.75% w/w water. The galenic compositions were formulated at 60 °C.
- Sample RBR (70%) + Filters: Oil/water emulsion was formulated containing a mixture of NEO PCL self-emulsifying base (Acofarma, Barcelona, Spain) and propylene glycol in water as the base formula. Ethylhexyl Methoxycinnamate, Butyl Methoxydibenzoylmethane and *Aspalathus linearis* extract (RBR Extract) were added to the propylene glycol-water phase prior to mixing with Neo PCL. The weight ratio of the ingredients in the final formula were 20% w/w Neo PCL, 5% w/w Propylene glycol, 4% w/w Ethylhexyl Methoxycinnamate, 2.5% w/w Butyl Methoxydibenzoylmethane, 1.75% w/w *Aspalathus linearis* extract (RBR Extract), and 66.75% w/w water. The galenic compositions were formulated at 60 °C.

- Sample RBR/RBG (RBR:RBG 70:30%) + Filters: Sample RBR (70%) + Filters: Oil/water emulsion was formulated containing a mixture of NEO PCL self-emulsifying base (Acofarma, Barcelona, Spain) and propylene glycol in water as the base formula. Ethylhexyl Methoxycinnamate, Butyl Methoxydibenzoylmethane and *Aspalathus linearis* extract (RBR Extract) were added to the propylene glycol-water phase prior to mixing with Neo PCL. The weight ratio of the ingredients in the final formula were 20% w/w Neo PCL, 5% w/w Propylene glycol, 4% w/w Ethylhexyl Methoxycinnamate, 2.5% Butyl Methoxydibenzoylmethane, 1.75% w/w *Aspalathus linearis* extract (RBR Extract), 0.75% w/w *Aspalathus linearis* extract (RBG Extract) and 66% w/w water. The galenic compositions were formulated at 60 °C.

Sample preparation for Example 7

Product 4

- **Phase A**

Under stirring, add Sodium Phytate and Succinic Acid in water. When ingredients are dissolved, add sodium hydroxide under stirring until pH is 7.4 -7.6. The solution becomes transparent. Add under stirring Sensiva™ (Phenylpropanol, Caprylyl Glycol and Tocopherol), and Tromethamine and heat phase to 75-80°C.

The weight ratio of the ingredients in the final formula is 0.1% w/w Sodium Phytate, 2% w/w Succinic Acid, 1% w/w Sensiva™ (Phenylpropanol, Caprylyl Glycol and Tocopherol), 1.22% w/w Tromethamine, 1% w/w Sodium hydroxide and 41.8133% w/w water.

- **Phase B**

Under rod stirring, add Finsolv® (C12-15 Alkyl Benzoate, Dipropylene Glycol Dibenzoate, PPG Stearyl Ether Benzoate), C13-15 Alkane, Phenethyl Benzoate, Cetiol® Ultimate (Undecane and Tridecane) and Ethylhexyl Salicylate, and heat to 75-80°C and mix with a homogenizer at 3000 rpm. Once the temperature is reached under high-speed mixer, add Butyl Methoxydibenzoylmethane, Bis-Ethylhexyloxyphenol Methoxyphenyl Triazine, Ethylhexyl Triazone, Sodium Polyacrylate, Disodium Cetearyl Sulfosuccinate, VP/Acrylates/Lauryl Methacrylate Copolymer, Acrylates/C 10-30 Alkyl Acrylate Crosspolymer, Tocopheryl Acetate, Pentaerythrityl Tetra-di-t-butyl Hydroxyhydrocinnamate, DOWSIL™ VM-2270 Aerogel (Silica Silylate and Aqua) and VALVANCE™ Touch 250 (Silica and Methicone).

The weight ratio of the ingredients in the final formula is 7% w/w Finsolv® (C12-15 Alkyl Benzoate, Dipropylene Glycol Dibenzoate, PPG-15 Stearyl Ether Benzoate), 0.5% w/w C13-15 Alkane, 1% w/w Cetiol® Ultimate (Undecane and Tridecane), 5% w/w Ethylhexyl Salicylate, 4.50% w/w Phenethyl Benzoate, 3% Butyl Methoxydibenzoylmethane, 3% w/w Bis-Ethylhexyloxyphenol Methoxyphenyl Triazine, 3% w/w Ethylhexyl Triazone, 0.2% w/w Sodium Polyacrylate, 0.5% w/w Disodium Cetearyl Sulfosuccinate, 0.5% w/w VP/Acrylates/Lauryl Methacrylate Copolymer, 0.3% w/w Acrylates/C 10-30 Alkyl Acrylate Crosspolymer, 0.1% w/w Tocopheryl Acetate, 0.1% w/w Pentaerythrityl Tetra-di-t-butyl Hydroxyhydrocinnamate, 0.5% w/w DOWSIL™ VM-2270 Aerogel (Silica Silylate and Aqua) and 3% w/w VALVANCE™ Touch 250 (Silica and Methicone).

- **Phase C**

The weight ratio of Novatext® Soft Focus 3 (Hydrogenated Vegetable Oil and Silica ingredients) in the final formula was 3% w/w.

- **Phase D**

Under rod stirring and without heating (Temperature <40°C), add water, niacidamide, Pixalia® (Propanediol, Water, Cleome Gynandra Leaf Extract), *Aspalathus linearis extract* (RBR Extract), *Aspalathus linearis extract* (RBG Extract), Polypodium leucotomos leaf extract, Propylene glycol, Tinosorb® M (Methylene Bis-Benzotriazolyl Tetramethylbutylphenol, Aqua, Decyl Glucoside, Propylene Glycol and Xanthan Gum), Parfum and Algaktiv® Genofix DUO (Water, Chlamydomonas Reinhardtii, extract, lecithin, Phenoxyethanol).

The weight ratio of the ingredients in the final formula is 3.0167% w/w water, 4% w/w niacidamide, 3% w/w Pixalia® Propanediol, Water, Cleome Gynandra Leaf Extract), 0.35% w/w *Aspalathus linearis extract* (RBR Extract), 0.15% w/w *Aspalathus linearis extract* (RBG Extract), 0.5 % Polypodium leucotomos leaf extract, 0.5% w/w Propylene Glycol, 4% w/w Tinosorb® M (Methylene Bis-Benzotriazolyl Tetramethylbutylphenol, Aqua, Decyl Glucoside, Propylene Glycol and Xanthan Gum), 0.5% w/w Parfum and 0.15% w/w Algaktiv® Genofix DUO (Water, Chlamydomonas Reinhardtii extract and lecithin).

- **Phase E**

The weight ratio of Sunhancer™ Eco SPF booster (Copernicia Cerifera (Carnauba) Wax and Oryza Sativa (Rice) Extract) is 1.5% w/w.

- **Emulsion**

Under high-shear mixing, add Phase A at a temperature of 75 - 80 °C. While maintaining a temperature of 75 - 80 °C turn off stirring, add phase B and emulsify Phase A and Phase B. Once emulsified, turn down the stirring speed and cool to 60°C. Add Phase C and cool to 40 - 50 °C and turn on high-shear mixing to emulsify. Turn off high-shear mixing, add Phase D and Phase E, turn on high-shear mixing. Turn off high-shear mixing and cool to 23 -27 °C.

Products 1, 2 and 3 follow the same manufacturing process. The ratio, by weight, of the ingredients is:

- **Product 1**

Phase A: 0.1% w/w Sodium Phytate, 2% w/w Succinic Acid, 1% w/w Sensiva™ (Phenylpropanol, Caprylyl Glycol and Tocopherol), 1.22% w/w Tromethamine, 1% w/w Sodium hydroxide and 41.8133% w/w water.
Phase B: 7% w/w Finsolv® (C12-15 Alkyl Benzoate, Dipropylene Glycol Dibenzoate), 0.5% w/w C13-15 Alkane, 1% w/w Cetiol® Ultimate (Undecane and Tridecane), 5% w/w Ethylhexyl Salicylate, 4.5% w/w Phenethyl Benzoate, 3% w/w Butyl Methoxydibenzoylmethane, 3% w/w Bis-Ethylhexyloxyphenol Methoxyphenyl Triazine, 3% w/w Ethylhexyl Triazone, 0.2% w/w Sodium Polyacrylate, 0.5% w/w Disodium Cetearyl Sulfosuccinate, 0.5% w/w VP/Acrylates/Lauryl Methacrylate Copolymer, 0.3% Acrylates/C 10-30 Alkyl Acrylate Crosspolymer, 0.1% w/w Tocopheryl Acetate, 0.1% w/w Pentaerythrityl Tetra-di-t-butyl Hydroxyhydrocinnamate, 0.5% w/w DOWSIL™ VM-2270 Aerogel (Silica Silylate and Aqua) and 3% w/w VALVANCE™ Touch 250 (Silica and Methicone).
Phase C: 3% w/w Novatext® Soft Focus 3 (Hydrogenated Vegetable Oil and Silica ingredients).
Phase D: 3.5167% w/w water, 4% w/w niacidamide, 3% w/w Pixalia® (Propanediol, Water, Cleome Gynandra Leaf Extract), 0.5 % Polypodium leucotomos leaf extract, 0.5% w/w Propylene Glycol, 4% w/w Tinosorb® M (Methylene Bis-Benzotriazolyl Tetramethylbutylphenol, Aqua, Decyl Glucoside, Propylene Glycol and Xanthan Gum), 0.5% w/w Parfum and 0.15% w/w Algaktiv® Genofix DUO (Water, Chlamydomonas Reinhardtii extract and lecithin).
Phase E: 1.5% w/w Sunhancer™ Eco SPF booster (Copernicia Cerifera (Carnauba) Wax and Oryza Sativa (Rice) Extract).

- **Product 2**

Phase A: 0.1% w/w Sodium Phytate, 2% w/w Succinic Acid, 1% w/w Sensiva™ (Phenylpropanol, Caprylyl Glycol and Tocopherol), 1.22% w/w Tromethamine, 1% w/w Sodium hydroxide and 41.8133% w/w water.
Phase B: 7% w/w Finsolv® (C12-15 Alkyl Benzoate, Dipropylene Glycol Dibenzoate, PPG-15 Stearyl Ether Benzoate), 0.5% w/w C13-15 Alkane, 1% w/w Cetiol® Ultimate (Undecane and Tridecane), 5% w/w Ethylhexyl Salicylate, 4.5% w/w Phenethyl Benzoate, 3% w/w Butyl Methoxydibenzoylmethane, 3% w/w Bis-Ethylhexyloxyphenol Methoxyphenyl Triazine, 3% w/w Ethylhexyl Triazone, 0.2% w/w Sodium Polyacrylate, 0.5% w/w Disodium Cetearyl Sulfosuccinate, 0.5% w/w VP/Acrylates/Lauryl Methacrylate Copolymer, 0.3% Acrylates/C10-30 Alkyl Acrylate Crosspolymer, 0.1% w/w Tocopheryl Acetate, 0.1% w/w Pentaerythrityl Tetra-di-t-butyl Hydroxyhydrocinnamate, 0.5% w/w DOWSIL™ VM-2270 Aerogel (Silica Silylate and Aqua) and 3% w/w VALVANCE™ Touch 250 (Silica and Methicone).
Phase C: 3% w/w Novatext® Soft Focus 3 (Hydrogenated Vegetable Oil and Silica ingredients).
Phase D: 3.1167% w/w water, 4% w/w niacidamide, 3% w/w Pixalia® (Propanediol, Water, Cleome Gynandra Leaf Extract), 0.35% w/w *Aspalathus linearis extract* (RBR Extract), 0.5 % Polypodium leucotomos leaf extract, 0.5% w/w Propylene Glycol, 4% w/w Tinosorb® M (Methylene Bis-Benzotriazolyl Tetramethylbutylphenol, Aqua, Decyl Glucoside, Propylene Glycol and Xanthan Gum), 0.5% w/w Parfum and 0.15% w/w Algaktiv® Genofix DUO (Water, Chlamydomonas Reinhardtii extract, lecithin, Phenoxyethanol).
Phase E: 1.5% w/w Sunhancer™ Eco SPF booster (Copernicia Cerifera (Carnauba) Wax and Oryza Sativa (Rice) Extract).

- **Product 3**

Phase A: 0.1% w/w Sodium Phytate, 2% w/w Succinic Acid, 1% w/w Sensiva™ (Phenylpropanol, Caprylyl Glycol and Tocopherol), 1.22% w/w Tromethamine, 1% w/w Sodium hydroxide and 41.8133% w/w water.

Phase B: 7% w/w Finsolv® (C12-15 Alkyl Benzoate, Dipropylene Glycol Dibenzoate, PPG-15 Stearyl Ether Benzoate), 0.5% w/w C13-15 Alkane, 1% w/w Cetiol® Ultimate (Undecane and Tridecane), 5% w/w Ethylhexyl Salicylate, 4.5% w/w Phenethyl Benzoate, 3% w/w Butyl Methoxydibenzoylmethane, 3% w/w Bis-Ethylhexyloxyphenol Methoxyphenyl Triazine, 3% w/w Ethylhexyl Triazone, 0.2% w/w Sodium Polyacrylate, 0.5% w/w Disodium Cetearyl Sulfosuccinate, 0.5% w/w VP/Acrylates/Lauryl Methacrylate Copolymer, 0.3% Acrylates/C10-30 Alkyl Acrylate Crosspolymer, 0.1% w/w Tocopheryl Acetate, 0.1% w/w Pentaerythrityl Tetra-di-t-butyl Hydroxyhydrocinnamate, 0.5% w/w DOWSIL™ VM-2270 Aerogel (Silica Silylate and Aqua) and 3% w/w VALVANCE™ Touch 250 (Silica and Methicone).

Phase C: 3% w/w Novatext® Soft Focus 3 (Hydrogenated Vegetable Oil and Silica ingredients).

Phase D: 3.3667% w/w water, 4% w/w niacidamide, 3% w/w Pixalia® (Propanediol, Water, Cleome Gynandra Leaf Extract), 0.15% w/w *Aspalathus linearis extract* (RBG Extract), 0.5 % Polypodium leucotomos leaf extract, 0.5% w/w Propylene Glycol, 4% w/w Tinosorb® M (Methylene Bis-Benzotriazolyl Tetramethylbutylphenol, Aqua, Decyl Glucoside, Propylene Glycol and Xanthan Gum), 0.5% w/w Parfum and 0.15% w/w Algaktiv® Genofix DUO (Water, Chlamydomonas Reinhardtii extract, lecithin, Phenoxyethanol).

Phase E: 1.5% w/w Sunhancer™ Eco SPF booster (Copernicia Cerifera (Carnauba) Wax and Oryza Sativa (Rice) Extract).

**Example 2. Photoprotective effect of RBR and RBG aqueous extracts separately**

*In vitro* culture of NHDF (Normal Human Dermal Fibroblast, Lonza, K3CC-2511) was standardized and grown in 96-well microtiter plates (Nunc 96 Well plate) until confluence (24 h). Cells were treated for 24 h with RBG and RBR extracts prepared according to Example 1, then washed, and UV irradiated (or not irradiated for control cells) using a lamp (Vilber Lourmat 312 nm VL-215 M). Plates were irradiated reaching an approximated total irradiation of 2.3 J/cm$^2$. After a period of recovery (overnight), cell survival was assessed by crystal blue staining (based on method (20)) and optical density (OD) reading (BMG Labtech, FLUOstar Optima). For data analysis, absorbance at 550 nm was normalized using first blank samples (no fibroblasts). Survival rates (in percentages) for UV-irradiated fibroblasts were calculated relative to nonirradiated fibroblasts in each group. To calculate the contribution of each treatment in induced-photoprotection percentages, the survival of the control non-treated UV-irradiated samples was assumed as "no induced-photoprotection", thus the increment between the survival rates in treated groups and the control non-treated group is expressed as "Photoprotection" in the bar graph. Then, the photoprotection rates shown for each treatment represent the average increment in survival rates relative to non-treated samples.
As intraexperimental control, the survival rate in non-treated samples was: 41.51% (close to LD50). The irradiation had a statistically significant effect in decreasing cell survival, with p-value ≤ 0.001, n = 36, validating the assay.

**Example 3. Synergic photoprotective effect of a combination of RBR and RBG extracts**

The experimental model used was an *in vitro* culture of NHDF as described in Example 2. We analyzed the contribution in photoprotection of a concentration of 0.175 mg/ml of aqueous RBR, 0.075 mg/ml of aqueous RBG, and a combination of both RBR:RBG (ratio 70:30 in weight) at a final concentration of 0.250 mg/ml. Figure 2 also included a bar (Sum RBR+RBG, indicated as S. RBR+RBG) representing the theoretical addition of RBR and RBG (70:30 at 0.250 mg/ml) calculated from the values obtained for RBR and RBG treatments (the two first bars shown in the graph). The tested concentrations were selected as being at the exponential phase of the dose/response curves performed in Figure 1, and therefore considered more adequate to explore induced variation in the biological process to study (photoprotection). As intraexperimental control, the survival rates were 59.03 % (close to LD50), and the effect of the irradiation was significant, with p-value ≤ 0.001 (n= 65).

## Example 4. Antioxidant capacity and chemical composition of the extracts

As in the main part of rooibos literature the biological effects are associated with the major components and their antioxidant capacity, we then characterized the aqueous RBG and RBR extracts used for examples 2 and 3.

The observed photoprotective effect does not correlate with the presence of major compounds in RBG and RBR aqueous extracts, as shown in Table 1.

Quantification was performed on an HPLC with a DAD detector with an Agilent Zorbax SB-C18, 1.8 μm, 100x4.6 mm column. The mobile phase consists of a gradient elution using the proportions of solvent A (2 % v/v acetic acid in MiliQ water) to solvent B (2% v/v acetic acid in acetonitrile) as follows: initial 90% A; 0 - 10 min, 87% A; 10 - 12 min, 79% A; 12-21 min, with a flow rate of 1 mL/min and the injection volume of 50 μL of samples and standards. The column temperature was maintained at 37°C throughout the analysis and the spectra were acquired in the 288 - 350 nm range. Chromatograms were plotted at 288 and 350 nm. Using these chromatographic conditions, it was possible to confirm the retention time of compounds by injection of the corresponding standard separately.

**Table 1**

| Compound | % w/w | |
| --- | --- | --- |
| | RBG | RBR |
| *Isoorientin* | 0.93 | 0.50 |
| *Orientin* | 1.03 | 0.26 |
| *Aspalathin* | 5.00 | 0.08 |
| *Vitexin* | 0.19 | 0.04 |
| *Hyperoside* | 0.02 | <0.01 |
| *Rutin* | 0.05 | 0.01 |
| *Isovitexin* | 0.19 | 0.11 |
| *Nothofagin* | 0.85 | 0.04 |
| *Quercitin* | 0.59 | 0.2 |
| *Luteoline* | 0.05 | 0.27 |

As shown in the table 1 and according to the literature, RBR extract showed a clear reduction in the main part of the compounds present in RBG previously associated with biological effects. RBR showed 62.5 times lower amount of Aspalathin, 21.25 times lower Nothofagin, 3.96 times lower Orientin, 1.86 times lower Isoorientin, 4.75 times less Vitexin and 1.7 times less Isovitexin, when compared to RBG.

The observed photoprotective effect did not correlate with the antioxidant capacity

As the main part of the biological effects ascribed to the extracts have been correlated with their antioxidant capacity, we performed FRAP analysis to evaluate the antioxidant competency of the extracts.

Samples were prepared as in Example 1. Each sample was placed in a Nunc 96 Well plate and diluted in FRAP reactive (TPTZ -2,4,6-trispyridil-s-triazine- 10 mM, $FeCl_3$ 20mM, and acetate buffer following 10:1:1, 37°C). Trolox C (6-hidroxy-2,5,7,8-tetramethylchromane-2-carboxylic acid) 0.2mM prepared in ethanol was used as a positive control. For FRAP quantification OD reading (BMG Labtech, FLUOstar Optima) set up at 593nm was performed.

As shown in Figure 3, RBR extract showed lower FRAP levels compared to RBG (Figure 3 b, c, and e). Using trolox as a reference antioxidant compound (Figure 3a), and as it is shown in Table 2, RBG showed 47.83% FRAP activity relative to trolox, while RBR showed 37.79% FRAP activity. Thus, RBR showed 21% less antioxidant activity compared to RBG.

Finally, the FRAP activity of a combination of RBR:RBG (in a proportion 70:30, mimicking Example 3) was measured. In addition, the theoretical contribution to FRAP by combining RBR and RBG in the mentioned rate assuming an additive effect was also calculated using the data measured for each of the extracts (Figures 3b and c corrected by the proportion of each ingredient in the combination -RBR values × 0.7, RBG values × 0.3-).

As shown in Figure 3f, theoretical and experimental FRAP capacity for the combination RBR:RBG showed highly similar levels.

The obtained values are pictured in the following Table 2.

**Table 2:** % of FRAP antioxidant capacities relative to Trolox, used as a reference antioxidant. Trolox values of reference were obtained by interpolating concentrations in Trolox linear equation (calculated in "a").

| | Relative to TROLOX (%) | | |
|---|---|---|---|
| Concentration mg/ml | RBR | RBG | RBR + RBG |
| 0.0005 | 41.57 | 55.97 | 38.68 |
| 0.0015 | 64.83 | 51.67 | 41.42 |
| 0.0035 | 23.27 | 50.60 | 39.97 |
| 0.0050 | 34.27 | 47.77 | 34.16 |
| AVERAGE | 37.79 | 47.83 | 35.97 |
| SEM ± | 6.73 | 3.49 | 2.55 |

## Example 5. Photoprotective effect of a combination of RBR and RBG extracts and EPL

We analyzed the contribution in photoprotection of a concentration of 0.5 mg/ml of a combination of EPL/RBR/RBG extracts prepared as in Example 1, tested in a proportion 50:35:15. The final concentration of each extract in the mixture is: EPL, 0.25 mg/ml; RBR, 0.175 mg/ml; and RBG, 0.075 mg/ml. To address the nature of the interaction of the different ingredients, they were included in the assay at the same concentrations as tested in combination (Figure 4). Treatments including the combination tested in Example 3 were also included for comparison: RBR:RBG (ratio 70:30 at final concentration 0.250 mg/ml, from 0.175 mg/ml RBR and 0.075 from RBG). The theoretical additive effect of EPL+RBR+RBG, EPL+RBR/RBG, and RBR+RBG, were also calculated and included in the graph of Figure 4 (shown as S. EPL+RBR+RBG, S. EPL+RBR/RBG, and S. RBR+RBG). As an intraexperimental control, the survival rate in non-treated samples was 57.9%, and the effect of the irradiation was significant, with p-value $\leq 0.001$, $n \geq 56$.

Figure 4 shows a synergic effect when comparing "EPL/RBR/RBG" vs "S. EPL+RBR+RBG" (statistically significant difference). Analyzing the nature of the synergy, the enhanced photoprotective effect seems to emerge from the combination of RBR/RBG; as the addition of the photoprotective effect from EPL to RBR/RBG ("S. EPL+RBR/RBG) was similar (not statistically distinguishable) to the experimental measures for EPL/RBR/RBG. This indicates a complementary effect of EPL in the combination of RBR/RBG. Conversely, there is a statistically significant increment in the photoprotection effect in RBR/RBG compared to "S. RBR+RBG", reinforcing the previously observed synergic phenomena for rooibos extract.

Therefore, the combination of the proposed ingredient (RBR:RBG) seems to perfectly complement the effect of other photoprotectant agents with similar MOA in the art when used as mixtures for photoprotection purposes.

## Example 6. Light filter and booster activity in combination with filters used in topical photoprotection

Different formulations in the form of topical sunscreens were tested. RBR, RBG, and their combination at a final concentration of 2.5% were included in a galenic formula similar to those used in sunscreens, containing +/- UVB and UVA organic filters. To address the nature of the interaction between the different ingredients, these were included in the assay at the same concentrations as tested in combination, RBR (70%), RBG (30%) and RBR:RBG (70:30%). The samples were prepared as explained in Example 1, with the following composition:

- Sample RBR (70%): Aqua, Cetearyl Ethylhexanoate, Ceresin, Lanolin, Sorbitan Sesquioleate, Stearyl Heptanoate, Propylene Glycol, Hydrogenated Castor Oil, Stearyl Caprylate, *Aspalathus linearis* extract (RBR Extract).
- Sample RBG (30%): Aqua, Cetearyl Ethylhexanoate, Ceresin, Lanolin, Sorbitan Sesquioleate, Stearyl Heptanoate, Propylene Glycol, Hydrogenated Castor Oil, Stearyl Caprylate, *Aspalathus linearis* extract (RBG Extract).
- Sample RBR/RBG (RBR:RBG 70:30%): Aqua, Cetearyl Ethylhexanoate, Ceresin, Lanolin, Sorbitan Sesquioleate, Stearyl Heptanoate, Propylene Glycol, Hydrogenated Castor Oil, Stearyl Caprylate, *Aspalathus linearis* extract (RBR Extract), *Aspalathus linearis* extract (RBG Extract).
- Sample Filters: Aqua, Cetearyl Ethylhexanoate, Ceresin, Lanolin, Sorbitan Sesquioleate, Stearyl Heptanoate, Propylene Glycol, Ethylhexyl Methoxycinnamate, Butyl Methoxydibenzoylmethane, Hydrogenated Castor Oil, Stearyl Caprylate..
- Sample RBG (30%) + Filters: Aqua, Cetearyl Ethylhexanoate, Ceresin, Lanolin, Sorbitan Sesquioleate, Stearyl Heptanoate, Propylene Glycol, Ethylhexyl Methoxycinnamate, Butyl Methoxydibenzoylmethane, Hydrogenated Castor Oil, Stearyl Caprylate, *Aspalathus linearis* extract (RBG Extract).
- Sample RBR (70%) + Filters: Aqua, Cetearyl Ethylhexanoate, Ceresin, Lanolin, Sorbitan Sesquioleate, Stearyl Heptanoate, Propylene Glycol, Ethylhexyl Methoxycinnamate, Butyl Methoxydibenzoylmethane, Hydrogenated Castor Oil, Stearyl Caprylate, *Aspalathus linearis* extract (RBR Extract).

- Sample RBR/RBG (RBR:RBG 70:30%) + Filters: Aqua, Cetearyl Ethylhexanoate, Ceresin, Lanolin, Sorbitan Sesquioleate, Stearyl Heptanoate, Propylene Glycol, Ethylhexyl Methoxycinnamate, Butyl Methoxydibenzoylmethane, Hydrogenated Castor Oil, Stearyl Caprylate, *Aspalathus linearis* extract (RBR Extract), *Aspalathus linearis* extract (RBG Extract).

Absorbance Properties

The spectral transmittance and absorbance of the different formulae were calculated (Figure 5a and b).

Briefly, the transmittance spectrum was determined by evenly spreading $1.3\,mg/cm^2$ of the product over $5\times5\,cm^2$ polymethyl methacrylate (PMMA) plate plaques (Schönberg, Hamburg, Germany). The plate had roughness simulating real skin relief, meeting the protocol indicated by ISO 24443:2020 standard. After 15 min in the dark, the sample was placed on the sensor (Ulbrich sphere type) of a Shimadzu UV-3101 PC spectrophotometer and illuminated with a 300 W Oriel solar simulator. The transmittance spectrum was analyzed at 1 nm intervals in the range 290-400 nm, referred to the spectral transmittance of the blank PMMA plate coated with glycerol.

Absorbance was calculated for each wavelength in the interval of 290-400 nm following the formula:

$$Absorbance = -Log\left(Transmittance\right)$$

As shown in Figure 5b, RBR (70%), RBG (30%), and RBR:RBG (70:30%) displayed certain absorbance in the UV spectral range. Relevant is that, even when showing a relatively low level of absorbance, the galenic formulations displayed enhanced filter properties when combined with the organic filters ethylhexyl Methoxycinnamate and Butyl Methoxydibenzoylmethane (Figure 5b).

Protection Factors of Sunscreen Formulations

The protection factors of each formulation were calculated *in vitro* using the measuring of the spectral transmittance in the UV range (290-400 nm), following the protocol indicated in ISO 24443:2012 for the analysis of protection factors for sunscreens.

Sun protection factor (SPF) was calculated as the protection potential against skin erythema, as an adaptation of the protocol described in Springsteen et al. 1999 (Springsteen A, Yurek R, Frazier M, Carr KF. In vitro measurement of sun protection factor of sunscreens by diffuse transmittance. Analytica Chimica Acta., 1999; 180(2-3): 155-164), using the following formula:

$$SPF = \frac{\int_{290}^{400} E_\lambda x\ \varepsilon_\lambda}{\int_{290}^{400} E_\lambda x\ \varepsilon_\lambda\ x\ T_\lambda}$$

In which SPF, sun protection factor; E, spectral irradiance of the solar simulator; $\varepsilon$, relative effectiveness for erythema; **T**, Transmittance of the sample and $\lambda$ wavelength.

As shown in Figure 5c, and in accordance with the patterns observed for the transmittance/absorbance, discrete levels of SPF were obtained for RBR (70%), RBG (30%) and RBR/RBG. Conversely, when combined with commercial organic filters, a statistical booster effect in SPF was observed in the galenic formulations including the proposed ingredients when compared with the SPF of the filters (Figure 5c). Similar performance was observed for UVA-PF (Figure 5d).

**Example 7. *In* vivo Sun Protection Factor (SPF) booster effect in topic products**

To determine the SPF boosting capability of RBR:RBG combination, in vivo SPF of a commercial sunscreen formulation was tested with added extracts of RBR (70 wt%), RBG (30 wt%) and RBR:RBG (70:30 wt%), in all cases at a final concentration of 0.5% w/w.

- Product 1 (Control Formulation): Aqua, C12-15 Alkl Benzoate, Ethylhexyl Salicylate, Phenethyl Benzoate, Silica, Niacinamide, Butyl Methoxydibenzoylmethane, Bis-Ethylhexyloxyphenol Methoxyphenyl Triazine, Ethylhexyl Triazone, Propanediol, Succinic Acid, Methylene Bis-BenzotriazonylTetramethylbutylphenol (nano), Hydrogenated Vegetable Oil, Copernicia Cerifera (Carnauba) Wax, Dipropylene Glycol Dibenzoate, Tromethamine, Sodium Hydroxide, *Polypodium leucotomos* Leaf Extract, Cleome Gynandra Leaf Extract, Chlamydomonas Reinhardtii Extract, Oryza Sative (Rice) Extract, Propylene Glycol, Tocopheryl Acetate, Tocopherol, Undecane, C13-15 Alkane, Disodium Cetearyl Sulfosuccinate, VP/Acrylates/Lauryl Methacrylate Copolymer, Silica Silylate, Phenylpropanol, PPG-15 Stearyl Ether Benzoate, Acrylates/C 10-30 Alkyl Acrylate Crosspolymer-1, Decyl Glucoside, Tridecane, Capryly Glycol, Sodium Polyacrylate, Sodium Phytate, Pentaerythrityl Tetra-Di-T-Butyl Hydroxydrocinnamate, Methicone, Lecithin, Xanthan Gum, Phenoxyethanol, Parfum.

- Product 2 (RBR 70%): Aqua, C12-15 Alkl Benzoate, Ethylhexyl Salicylate, Phenethyl Benzoate, Silica, Niacinamide, Butyl Methoxydibenzoylmethane, Bis-Ethylhexyloxyphenol Methoxyphenyl Triazine, Ethylhexyl Triazone, Propanediol, Succinic Acid, Methylene Bis-Benzotriazonyl etramethylbutylphenol (nano), Hydrogenated Vegetable Oil, Copernicia Cerifera Carnauba) Wax, Dipropylene Glycol Dibenzoate, Tromethamine, Sodium Hydroxide, *Polypodium leucotomos* Leaf Extract, Cleome Gynandra Leaf Extract, Chlamydomonas Reinhardtii Extract, Oryza Sative (Rice) Extract, Propylene Glycol, Tocopheryl Acetate, Tocopherol, Undecane, C13-15 Alkane, Disodium Cetearyl Sulfosuccinate, VP/Acrylates/Lauryl Methacrylate Copolymer, Silica Silylate, Phenylpropanol, PPG-15 Stearyl Ether Benzoate, Acrylates/C 10-30 Alkyl Acrylate Crosspolymer-1, Decyl Glucoside, Tridecane, Capryly Glycol, Sodium Polyacrylate, Sodium Phytate, Pentaerythrityl Tetra-Di-Tbutyl Hydroxydrocinnamate, Methicone, Lecithin, Xanthan Gum, Phenoxyethanol, *Aspalathus linearis* Extract (RBR Extract), Parfum.

- Product 3 (RBG 30%): Aqua, C12-15 Alkl Benzoate, Ethylhexyl Salicylate, Phenethyl Benzoate, Silica, Niacinamide, Butyl Methoxydibenzoylmethane, Bis-Ethylhexyloxyphenol Methoxyphenyl Triazine, Ethylhexyl Triazone, Propanediol, Succinic Acid, Methylene Bis-Benzotriazonyl etramethylbutylphenol(nano), Hydrogenated Vegetable Oil, Copernicia Cerifera Carnauba) Wax , Dipropylene Glycol Dibenzoate, Tromethamine, Sodium Hydroxide, *Polypodium leucotomos* Leaf Extract, Cleome Gynandra Leaf Extract, Chlamydomonas Reinhardtii Extract, Oryza Sative (Rice) Extract, Propylene Glycol, Tocopheryl Acetate, Tocopherol, Undecane, C13-15 Alkane, Disodium Cetearyl Sulfosuccinate, VP/Acrylates/Lauryl Methacrylate Copolymer, Silica Silylate, Phenylpropanol, PPG-15 Stearyl Ether Benzoate, Acrylates/C 10-30 Alkyl Acrylate Crosspolymer-1, Decyl Glucoside, Tridecane, Capryly Glycol, Sodium Polyacrylate, Sodium Phytate, Pentaerythrityl Tetra-Di-Tbutyl Hydroxydrocinnamate, Methicone, Lecithin, Xanthan Gum, Phenoxyethanol, *Aspalathus linearis* Extract (RBG Extract), Parfum.

- Product 4 (RBR:RBG 70:30): Aqua, C12-15 Alkl Benzoate, Ethylhexyl Salicylate, Phenethyl Benzoate, Silica, Niacinamide, Butyl Methoxydibenzoylmethane, Bis-Ethylhexyloxyphenol Methoxyphenyl Triazine, Ethylhexyl Triazone, Propanediol, Succinic Acid, Methylene Bis-Benzotriazonyl Tetramethylbutylphenol (nano), Hydrogenated Vegetable Oil, Copernicia Cerifera (Carnauba) Wax, Dipropylene Glycol Dibenzoate, Tromethamine, Sodium Hydroxide, *Polypodium leucotomos* Leaf Extract, Cleome Gynandra Leaf Extract, Chlamydomonas Reinhardtii Extract, Oryza Sative (Rice) Extract, Propylene Glycol, Tocopheryl Acetate, Tocopherol, Undecane, C13-15 Alkane, Disodium Cetearyl Sulfosuccinate, VP/Acrylates/Lauryl Methacrylate Copolymer, Silica Silylate, Phenylpropanol, PPG-15 Stearyl Ether Benzoate, Acrylates/C 10-30 Alkyl Acrylate Crosspolymer-1, Decyl Glucoside, Tridecane, Capryly Glycol, Sodium Polyacrylate, Sodium Phytate, Pentaerythrityl Tetra-Di-Tbutyl Hydroxydrocinnamate, Methicone, Lecithin, Xanthan Gum, Phenoxyethanol, *Aspalathus linearis* Extract (RBR Extract), *Aspalathus linearis* Extract (RBG Extract), Parfum.

*In vivo* SPF determination for each formulation was carried out following the international Standard - ISO 24444:2019 protocol.

General Procedure

The method employs a solar simulator with a xenon arc lamp with a defined and known output. A section of the skin (on the back) for each subject is exposed to UV light without any protection, while another area (different than the first one) is exposed following product application.

To validate the procedure, an additional section of the back is exposed to UV light after the application of a reference standard product with a verified SPF factor (medium SPF, product reference 63.1).

To determine the sun protection factor, an incremental series of late erythematous responses are induced in small secondary areas of the skin. These responses are visually assessed according to the presence of redness in a period between 16 h and 24 h after exposure to UV radiation. The variables obtained thereby were the minimum erythematous dose for unprotected skin (MEDu) and the minimum erythematous dose for protected skin (MEDp) after the application of a sunscreen product.

The minimum erythematous dose (MED) is expressed in terms of energy ($mJ \times cm^2$), or time units (seconds after UV light exposition).

Tested Area

- The back is the anatomical region selected to perform the test.
- The product is applied in a minimum area of 30 cm$^2$ and a maximum area of 60 cm$^2$.
- Minimum distance between the edges of adjacent test sites: 1 cm.
- The positions of the products and standards should be randomly distributed on the backs of all subjects, in order to reduce errors derived from anatomical differences in the skin.
- All products must be homogeneous and must be shaken before being weighed, to ensure uniform dispersion. A latex, nitrile, etc. finger should be used for the application, except in cases where the use of finger protection interferes with the application, then it is applied with the bare finger and a maximum of 2.1 mg/cm$^2$ (additional 5%) is weighed.
- Tested product and reference product were taken by micro pipetting and applied at a rate of 2.00 ±0.05 mg/cm$^2$.

Sun Protection Factor Calculations and Statistics

The individual sun protection factor (SPFi) for each subject tested is determined from the measurements collected from the same individual, according to the following formula, and expressed to one decimal place.

$$SPFi = MEDp/\ Medu$$

The SPF of the product is the arithmetic mean of the individual SPFi values obtained from the total number of subjects with valid results, expressed to a decimal place.

$$SPF = (\Sigma\ SPFi)\ /\ n$$

Their standard deviation is given according to the formula:

$$s = \sqrt{[(\Sigma(SPFi^2) - (\Sigma(SPFi)^2/\ n))\ /\ (n-1)]}$$

and the 95% confidence interval (95% CI) of the mean SPF should be expressed as:

$$95\%\ IC = [\ FPS - c\ ;\ FPS + c\ ]$$

from which c is calculated = (value of t). SEM = t · s / $\sqrt{\Sigma\ n}$
where:

SEM = standard error of the mean

$$s = \text{standard deviation} = \sqrt{[(\Sigma(FPSi^2) - (\Sigma(FPSi)^2/\ n))\ /\ (n-1))]}$$

n = total number of subjects whose results are exploitable
t = t-value in the two-sided Student's t-distribution table with probability p = 0.05 and number of degrees of freedom df = n-1

According to this method, a minimum of 10 valid results is sufficient if the statistical criterion is met. As a statistical criterion for tested products and sunscreens of references (standards), the 95% confidence interval (CI) should be included in a range of ±17% of the mean SPF.

**Table 3:** *In vivo* SPF booster effect.

| | Product 1 Commercial Formulation | Product 2 Commercial Formulation + 0.5% RBR (70%) | Product 3 Commercial Formulation + 0.5% RBG (30%) | Product 4 Commercial Formulation + 0.5% RBR:RBG (70:30%) |
|---|---|---|---|---|
| SPF | 57.9 | 59.6 | 64.7 | 66.3 |
| Standard deviation | 6.9 | 7.4 | 7.7 | 7.7 |
| C | 4.9 | 8.3 | 5.5 | 5.5 |
| IC (%) | 8 | 9 | 8 | 8 |
| *Where, c = t * s / √n with: t = t-value of Student's t-distribution for 95% Confidence Interval | | | | |

As shown in Table 3, when 0.5% of RBR:RGB (70:30%) is included in a commercial galenic formulation (Product 4), an SPF-enhancing effect is observed compared to the same product without the combination (Product 1). The booster effect of a 0.5% of RBR:RGB (70:30%) increases in a 14.6% the SPF *in vivo* value.

## Example 7. Topical dosage forms

Four models of topical dosage forms are herein described:

Commercial formula A

| | %w/w range | |
|---|---|---|
| | % | % |
| OCTOCRYLENE | 0.1000 | 50.0000 |
| ETHYLHEXYL SALICYLATE | 0.1000 | 25.0000 |
| BUTYL METHOXYDIBENZOYLMETHANE | 0.1000 | 20.0000 |
| DIETHYLAMINO HYDROXYBENZOYL HEXYL BENZOATE | 0.1000 | 10.0000 |
| PHENYLBENZIMIDAZOLE SULFONIC ACID | 0.1000 | 15.0000 |
| ETHYLHEXYL TRIAZONE | 0.0500 | 2.5000 |
| TRIS-BIPHENYL TRIAZINE(NANO). DECYL GLUCOSIDE. BUTYLENE GLYCOL. DISODIUM PHOSPHATE. XANTHAN GUM. AQUA (50: 7.5: 0.4: 0.4: 0.3 : 41.40) | 0.0100 | 0.5000 |
| STYRENE/ACRYLATES COPOLYMER & PEG-8 LAURATE & AQUA | 0.1000 | 5.0000 |
| AQUA | 0.0100 | 95.0000 |
| PHENETHYL BENZOATE | 0.2000 | 10.0000 |
| BUTYLENE GLYCOL COCOATE | 0.0010 | 2.0000 |
| SILICA & METHICONE | 0.2000 | 10.0000 |
| POLYMETHYLSILSESQUIOXANE | 0.1000 | 5.0000 |
| VP/ACRYLATES/LAURYL METHACRYLATE COPOLYMER | 0.0500 | 2.5000 |
| SODIUM ACRYLATES CROSSPOLYMER-2 | 0.0250 | 1.2500 |
| DIMETHICONE | 0.2500 | 12.5000 |
| CELLULOSE & MAGNESIUM STEARATE | 0.0010 | 20000 |
| C20-22 ALKYL PHOSPHATE & C20-22 ALCOHOLS & AQUA | 0.1500 | 7.5000 |
| ACRYLATES/C10-30 ALKYL ACRYLATE CROSSPOLYMER | 0.0100 | 0.5000 |

(continued)

|  | %w/w range | |
| --- | --- | --- |
|  | % | % |
| BUTYLENE GLYCOL | 0.3000 | 15.0000 |
| PHENYLPROPANOL & PROPANEDIOL & CAPRYLYL GLYCOL & TOCOPHEROL | 0.1500 | 7.5000 |
| DISODIUM EDTA | 0.0100 | 0.5000 |
| SODIUM PHYTATE | - | - |
| TOCOPHERYL ACETATE | 0.0100 | 0.5000 |
| BHT | 0.0050 | 0.2500 |
| PEG-8 & TOCOPHEROL & ASCORBYL PALMITATE & ASCORBIC ACID & CITRIC ACID | - | - |
| ARGININE | 0.2650 | 13.2500 |
| PARFUM & LINALOOL | 0.0050 | 0.2500 |
| AQUA & GLYCERIN & TREHALOSE & UREA & SERINE & PENTYLENE GLYCOL & GLYCERYL POLYACRYLATE & ALGIN & CAPRYLYL GLYCOL & SODIUM HYALURONATE & PULLULAN & DISODIUM PHOSPHATE & POTASSIUM PHOSPHATE | 0.3000 | 15.0000 |
| PHYSALIS ANGULATA EXTRACT & CAPRYLIC/CAPRIC TRIGLYCERIDE & TOCOPHEROL | 0.0200 | 1.0000 |
| AQUA & PLANKTON EXTRACT & LECITHIN & PHENOXYETHANOL | 0.0125 | 0.6250 |
| AQUA & OXOTHIAZOLIDINE & BUTYLENE GLYCOL & SODIUM BENZOATE | 0.0100 | 0.5000 |
| AQUA & CHLAMYDOMONAS REINHARDTII EXTRACT & LECITHIN & PHENOXYETHANOL | 0.0075 | 0.3750 |
| AQUA & PLANKTON EXTRACT & LECITHIN & PHENOXYETHANOL | 0.0050 | 0.2500 |
| AQUA & MELANIN & PENTYLENE GLYCOL & ETHYLHEXYLGLYCERIN | 0.0001 | 0.0050 |
| POLYPODIUM LEUCOTOMOS LEAF EXTRACT | 0.0001 | 10.0000 |
| ASPALATHUS LINEARIS EXTRACT | 0.0001 | 100000 |

- Commercial formula B

|  | %w/w range | |
| --- | --- | --- |
|  | % | % |
| CYCLOPENTASILOXANE & TITANIUM DIOXIDE (NANO) & POLYGLYCERYL-3 POLYDIMETHYLSILOXYETHYL DIMETHICONE & ALUMINUM HYDROXIDE & STEARIC ACID | 0.1000 | 50.0000 |
| ZINC OXIDE & TRIETHOXYCAPRYLYLSILANE | 0.1000 | 20.0000 |
| ETHYLHEXYL SALICYLATE | 0.0500 | 20.0000 |
| BIS-ETHYLHEXYLOXYPHENOL METHOXYPHENYL TRIAZINE | 0.1200 | 4.8000 |
| ETHYLHEXYL TRIAZONE | 0.0700 | 2.8000 |
| DIETHYLAMINO HYDROXYBENZOYL HEXYL BENZOATE | 0.1200 | 4.8000 |
| TRIS-BIPHENYL TRIAZINE & AQUA & DECYL GLUCOSIDE & BUTYLENE GLYCOL & DISODIUM PHOSPHATE & XANTHAN GUM | 0.0000 | 0.0000 |
| STYRENE/ACRYLATES COPOLYMER & PEG-8 LAURATE & AQUA | 0.1800 | 7.2000 |
| AQUA | 0.1000 | 95.0000 |
| PHENETHYL BENZOATE | 0.2500 | 100000 |

(continued)

|  | %w/w range | |
|---|---|---|
|  | % | % |
| DIISOPROPYL ADIPATE | 0.4500 | 18.0000 |
| DIETHYLHEXYL CARBONATE | 0.2500 | 10.0000 |
| ISONONYL ISONONANOATE | 0.1500 | 6.0000 |
| ISODODECANE | 1.2000 | 48.0000 |
| ISODODECANE & DISTEARDIMONIUM HECTORITE & PROPYLENE CARBONATE | 0.6000 | 24.0000 |
| TRISILOXANE | 0.5000 | 20.0000 |
| CAPRYLYL METHICONE | 0.3500 | 140000 |
| BUTYLENE GLYCOL | 0.2900 | 11.6000 |
| PEG-9 POLYDIMETHYLSILOXYETHYL DIMETHICONE | 0.2000 | 8.0000 |
| CETYL PEG/PPG-10/1 DIMETHICONE | 0.2800 | 11.2000 |
| PHENYLPROPANOL & PROPANEDIOL & CAPRYLYL GLYCOL & TOCOPHEROL | 0.1500 | 6.0000 |
| POLYMETHYLSILSESQUIOXANE | 0.1200 | 4.8000 |
| MAGNESIUM SULFATE | 0.0500 | 2.0000 |
| PARFUM & LINALOOL | 0.0250 | 1.0000 |
| TOCOPHERYL ACETATE | 0.0200 | 0.8000 |
| CI 77492 & TRIETHOXYCAPRYLYLSILANE | 0.0173 | 0.6920 |
| DISODIUM EDTA | 0.0000 | 0.0000 |
| SODIUM PHYTATE | 0.0100 | 0.4000 |
| AQUA. TRISODIUM ETHYLENEDIAMINE DISUCCINATE | 0.0000 | 0.0000 |
| PEG-8 & TOCOPHEROL & ASCORBYL PALMITATE & ASCORBIC ACID & CITRIC ACID | 0.0050 | 0.2000 |
| CI 77491. TRIETHOXYCAPRYLYLSILANE | 0.0027 | 0.1080 |
| CI 77499. TRIETHOXYCAPRYLYLSILANE | 0.0025 | 0.1000 |
| SILICA. MACADAMIA INTEGRIFOLIA SEED OIL. TRIHYDROXYSTEARIN | 0.0000 | 0.0000 |
| POLYGLYCERYL-2 OLEATE. POLYHYDROXYSTEARIC ACID. POLYGLYCERYL-2 STEARATE | 0.0000 | 0.0000 |
| DISTEARDIMONIUM HECTORITE | 0.0000 | 0.0000 |
| HYDROGENATED VEGETABLE OIL. ISODODECANE | 0.0000 | 0.0000 |
| NIACINAMIDE | 0.3000 | 12.0000 |
| AQUA & GLYCERIN & TREHALOSE & UREA & SERINE & PENTYLENE GLYCOL & GLYCERYL POLYACRYLATE & ALGIN & CAPRYLYL GLYCOL & SODIUM HYALURONATE & PULLULAN & DISODIUM PHOSPHATE & POTASSIUM PHOSPHATE | 0.1000 | 4.0000 |
| AQUA & CHLAMYDOMONAS REINHARDTII EXTRACT & LECITHIN & PHENOXYETHANOL | 0.0200 | 0.8000 |
| PUNICA GRANATUM FRUIT EXTRACT | 0.0005 | 0.0200 |
| POLYPODIUM LEUCOTOMOS LEAF EXTRACT | 0.0001 | 100000 |
| ASPALATHUS LINEARIS EXTRACT | 0.0001 | 100000 |

- Commercial formula C

| | %w/w range | |
|---|---|---|
| | % | % |
| AQUA | 0.1000 | 95.0000 |
| C12-15 ALKYL BENZOATE | 0.5250 | 21.0000 |
| ARGININE | 0.4520 | 18.0800 |
| PHENYLBENZIMIDAZOLE SULFONIC ACID | 0.4000 | 16.0000 |
| DISODIUM PHENYL DIBENZIMIDAZOLE TETRASULFONATE | 0.4000 | 16.0000 |
| BIS-ETHYLHEXYLOXYPHENOL METHOXYPHENYL TRIAZINE | 0.3000 | 12.0000 |
| TITANIUM DIOXIDE (NANO) | 0.2952 | 11.8080 |
| HEXYLENE GLYCOL | 0.2250 | 90000 |
| C14-22 ALCOHOLS | 0.2000 | 8.0000 |
| C12-20 ALKYL GLUCOSIDE | 0.2000 | 8.0000 |
| DIETHYLAMINO HYDROXYBENZOYL HEXYL BENZOATE | 0.2000 | 8.0000 |
| NYLON-12 | 0.2000 | 8.0000 |
| MYRISTYL ALCOHOL | 0.1500 | 6.0000 |
| DIPROPYLENE GLYCOL DIBENZOATE | 0.1400 | 5.6000 |
| CETEARETH-25 | 0.1050 | 4.2000 |
| DIETHYLHEXYL BUTAMIDO TRIAZONE | 0.1000 | 4.0000 |
| PHENOXYETHANOL | 0.1000 | 4.0000 |
| POLYPODIUM LEUCOTOMOS EXTRACT | 0.1000 | 40000 |
| PHYSALIS ANGULATA EXTRACT | 0.0150 | 0.6000 |
| CAPRYLYL GLYCOL | 0.0700 | 2.8000 |
| MELANIN | 0.0200 | 0.8000 |
| MYRISTYL GLUCOSIDE | 0.0500 | 2.0000 |
| ETHYLHEXYL TRIAZONE | 0.0500 | 2.0000 |
| CAPRYLYL METHICONE | 0.0500 | 2.0000 |
| HYDROLYZED WHEAT PROTEIN/PVP CROSSPOLYMER | 0.0500 | 2.0000 |
| GLYCERIN | 0.0400 | 1.6000 |
| 3-O-ETHYL ASCORBIC ACID | 0.0200 | 0.8000 |
| ALUMINA | 0.0396 | 1.5840 |
| PPG-15 STEARYL ETHER BENZOATE | 0.0350 | 1.4000 |
| DISODIUM ETHYLENE DICOCAMIDE PEG-15 DISULFATE | 0.0450 | 1.8000 |
| CAMELLIA SINENSIS EXTRACT | 0.0200 | 0.8000 |
| ETHYLHEXYLGLYCERIN | 0.0300 | 1.2000 |
| TOCOPHERYL ACETATE | 0.0100 | 0.4000 |
| XANTHAN GUM | 0.0200 | 0.8000 |
| PHYTOSPHINGOSINE HCL | 0.0050 | 0.2000 |
| PLANKTON EXTRACT | 0.0013 | 0.0520 |
| ETHYL LAUROYL ARGINATE HCL | 0.0100 | 0.4000 |
| PANTHENYL TRIACETATE | 0.0100 | 0.4000 |
| PARFUM | 0.0150 | 0.6000 |

(continued)

| | %w/w range | |
|---|---|---|
| | % | % |
| ALÉRGENOS DECLARABLES: LINALOOL 0.01815% | 0.0000 | 0.0000 |
| SIMETHICONE | 0.0108 | 0.4320 |
| OXOTHIAZOLIDINE | 0.0010 | 0.0400 |
| DISODIUM EDTA | 0.0100 | 0.4000 |
| SILICA | 0.0100 | 0.4000 |
| ETHYL LINOLEATE | 0.0052 | 0.2080 |
| CAPRYLIC/CAPRIC TRIGLYCERIDE | 0.0050 | 0.2000 |
| OLEYL ALCOHOL | 0.0046 | 0.1840 |
| BUTYLENE GLYCOL | 0.0020 | 0.0800 |
| PROPYLENE GLYCOL | 0.0468 | 1.8720 |
| LECITHIN | 0.0001 | 0.0040 |
| SODIUM BENZOATE | 0.0001 | 0.0040 |
| TOCOPHEROL | 0.0002 | 0.0080 |
| ASPALATHUS LINEARIS EXTRACT | 0.0001 | 100000 |

- Commercial formula D

| | %w/w range | |
|---|---|---|
| | % | % |
| AQUA | 0.1000 | 95.0000 |
| DIMETHICONE | 0.1000 | 70.0000 |
| ISODODECANE | 0.1000 | 70.0000 |
| C12-15 ALKYL BENZOATE | 0.8846 | 35.3829 |
| HYDROGENATED POLYDECENE | 0.8000 | 32.0000 |
| TITANIUM DIOXIDE (NANO) | 0.7000 | 28.0014 |
| ZINC OXIDE (NANO) | 0.6003 | 24.0106 |
| BUTYLOCTYL SALICYLATE | 0.3144 | 12.5770 |
| ALUMINA | 0.1409 | 5.6371 |
| STYRENE/ACRYLATES COPOLYMER | 0.1250 | 50000 |
| PHENETHYL BENZOATE | 0.1000 | 4.0000 |
| PEG-9 POLYDIMETHYLSILOXYETHYL DIMETHICONE | 0.1000 | 4.0000 |
| NYLON-12 | 0.1000 | 4.0000 |
| POLYPODIUM LEUCOTOMOS EXTRACT | 0.1000 | 40000 |
| FERULIC ACID | 0.0005 | 0.0200 |
| CAFFEIC ACID | 0.0001 | 0.0040 |
| PHYSALIS ANGULATA EXTRACT | 0.0150 | 0.6000 |
| CAPRYLYL GLYCOL | 0.0451 | 1.8036 |
| MELANIN | 0.0001 | 0.0040 |

(continued)

| | %w/w range | |
|---|---|---|
| | % | % |
| CAMELLIA SINENSIS EXTRACT | 0.0002 | 0.0080 |
| PLANKTON EXTRACT | 0.0013 | 0.0500 |
| 3-O-ETHYL ASCORBIC ACID | 0.0200 | 0.8000 |
| ISOHEXADECANE | 0.0938 | 3.7500 |
| HYDROGEN DIMETHICONE | 0.0709 | 2.8370 |
| PHENYLPROPANOL | 0.0675 | 2.7000 |
| POLYHYDROXYSTEARIC ACID | 0.0553 | 2.2106 |
| MAGNESIUM SULFATE | 0.0500 | 20000 |
| TOCOPHERYL ACETATE | 0.0500 | 2.0000 |
| DIMETHICONE/PEG-10/15 CROSSPOLYMER | 0.0480 | 1.9200 |
| PROPANEDIOL | 0.0375 | 1.5000 |
| TRIETHOXYCAPRYLYLSILANE | 0.0290 | 1.1614 |
| PROPYLENE CARBONATE | 0.0063 | 0.2500 |
| CAPRYLIC/CAPRIC TRIGLYCERIDE | 0.0050 | 0.1999 |
| PANTHENYL TRIACETATE | 0.0025 | 0.1000 |
| BUTYLENE GLYCOL | 0.0020 | 0.0800 |
| PROPYLENE GLYCOL | 0.0468 | 1.8720 |
| ARGININE | 0.0006 | 0.0240 |
| ETHYL LINOLEATE | 0.0013 | 0.0520 |
| OLEYL ALCOHOL | 0.0012 | 0.0460 |
| PENTYLENE GLYCOL | 0.0010 | 0.0400 |
| OXOTHIAZOLIDINE | 0.0010 | 0.0400 |
| DIPROPYLENE GLYCOL | 0.0010 | 0.0400 |
| SODIUM CITRATE | 0.0004 | 0.0160 |
| PALMITOYL HYDROXYPROPYLTRIMONIUM AMYLOPECTIN/GLYCERIN CROSSPOLYMER | 0.0002 | 0.0080 |
| 1.2-HEXANEDIOL | 0.0001 | 0.0040 |
| LECITHIN | 0.0001 | 0.0040 |
| SODIUM BENZOATE | 0.0001 | 0.0020 |
| TOCOPHEROL | 0.0001 | 0.0037 |
| HYDROGENATED LECITHIN | 0.0000 | 0.0004 |
| PHENOXYETHANOL | 0.0001 | 0.0020 |
| ASPALATHUS LINEARIS EXTRACT | 0.0001 | 100000 |

## Example 9. Oral dosage forms

Four models of oral dosage forms are herein described:

- Commercial formula A

|  | %w/w range | |
| --- | --- | --- |
|  | % | % |
| POLYPODIUM LEUCOTOMOS EXTRACT | 0.1000 | 90.0000 |
| ASPALATHUS LINEARIS EXTRACT | 0.1000 | 90.0000 |
| N-ACETYLCISTEINE | 0.1000 | 90.0000 |
| GLUCO-OLIGOSACCHARIDES | 0.0100 | 30.0000 |
| INULINE | 0.0100 | 30.0000 |
| ASCORBIC ACID | 0.0100 | 30.0000 |
| NATURAL ORANGE FLAVOR | 0.0100 | 100000 |
| NIACINAMIDE | 0.0100 | 90.0000 |
| VITAMIN E 50% (DL-ALPHA TOCOPHENYL ACETATE | 0.0100 | 90.0000 |
| NEOHESPERIDINE DC (5-959) | 0.0001 | 50.0000 |
| HYDROXYPROPYL-METHYL CELLULOSE | 0.1000 | 30.0000 |
| IRON OXIDES (E-172) | 0.1000 | 30.0000 |
| WATER | 0.1000 | 99.0000 |

- Commercial formula B

|  | %w/w range | |
| --- | --- | --- |
|  | % | % |
| POLYPODIUM LEUCOTOMOS LEAF EXTRACT | 0.1000 | 90.0000 |
| ASPHALATHUS LINEARIS EXTRACT | 0.1000 | 90.0000 |
| EMDEX | 0.1000 | 90.0000 |
| NATURAL ORANGE FLAVOR | 0.0100 | 30.0000 |
| CITRIC ACID MONOHYDRTE | 0.0100 | 30.0000 |
| NEOHESPERIDINE | 0.0001 | 50.0000 |
| MAGNESIUM STEARATE | 0.0100 | 30.0000 |

- Commercial formula C

|  | %w/w range | |
| --- | --- | --- |
|  | % | % |
| POLYPODIUM LEUCOTOMO LEAF EXTRACT | 0.1000 | 90.0000 |
| ASPALATHUS LINEARIS EXTRACT | 0.1000 | 90.0000 |
| MALTODEXTRIN | 0.1000 | 90.0000 |
| CAMELIA SINENSIS EXTRACT | 0.1000 | 90.0000 |
| BETACAROTENE 10 % | 0.1000 | 90.0000 |
| MAGNESIUM SALTS OF FATTY ACID (e-470B) | 0.0100 | 50.0000 |
| HYDROXYPROPYL-METHYL CELLULOSE | 0.1000 | 90.0000 |
| POLYETHYLENE GLYCOL 400 | 0.1000 | 90.0000 |

- Commercial formula D

| | % w/w range | |
|---|---|---|
| | % | % |
| POLYPODIUM LEUCOTOMOS LEAF EXTRACT | 0.1000 | 90.0000 |
| ASPHALATHUS LINEARIS EXTRACT | 0.1000 | 90.0000 |
| ISOMALTOSE | 0.1000 | 95.0000 |
| NATURAL ORANGE FLAVOR | 0.0010 | 30.0000 |
| CITRIC ACID MONOHYDRTE | 0.0010 | 50.0000 |
| SUCRALOSE | 0.0010 | 30.0000 |

**Claims**

1. A combination comprising a green *Aspalathus linearis* (RBG) extract and a red *Aspalathus linearis* (RBR) extract, for use in the prophylaxis or treatment of a sun radiation-induced disease or disorder in a subject.

2. A combination for use according to claim 1, wherein said RBG and/or RBR extract is a water extract.

3. A combination for use according to claim 1 or 2, wherein said RBR and RBG extracts are in proportion of RBR:RBG 70:30 wt%.

4. A combination for use according to any one of claims 1 to 3, wherein said prophylaxis or treatment is photoprotection of a cell of the skin, mucous membranes, scalp and/or hair, or healing and/or re-epithelialization of wounds.

5. A combination for use according to any one of claims 1 to 4, wherein said disease or disorder is cell damage, photoaging, thermal stress, oxidative stress, osmotic shock, inflammation, hypoxia, exposure to pollutants, lack of nourishment and lack of hydration, epidermolysis bullosa, a skin pigmentation disorder, vitiligo, reactive or dry skin, psoriasis, or acne.

6. Use of a combination of an extract of green *Aspalathus linearis* (RBG) and an extract of red *Aspalathus linearis* (RBR), in the non-therapeutical treatment or care of skin, mucous membranes, scalp and/or hair.

7. Use according to claim 6, in which said non-therapeutical treatment of the skin is the prevention of wrinkles.

8. Use according to claim 6, in which said non-therapeutical treatment of the skin is a regulation of pigmentation.

9. Use according to claim 8, in which said regulation of pigmentation is a whitening or lightening of scars.

10. Use according to claim 6, in which said care of the skin is a postponement and/or prevention of signs of photoaging of the skin.

11. Use according to claim 6, in which said care of the hair is hair gloss, hair density, oiliness, sebum production, glossiness, or hair coat loss.

12. A pharmaceutical, nutraceutical or cosmetic composition comprising a combination of an RBG and an RBR extracts, and at least one pharmaceutical, nutraceutical or cosmetical acceptable excipient or adjuvant.

13. A pharmaceutical, nutraceutical or cosmetic composition according to claim 12, comprising an EPL extract.

14. A pharmaceutical or nutraceutical composition according to claim 12 or 13, in which said composition is for oral administration.

15. An oral pharmaceutical or nutraceutical composition according to claim 14, in which said combination of RBR and RBG extracts is present in an amount of 1 to 90 wt%, or 10 to 50 wt%, or 20 to 30 wt%, or of 25 wt% of the total weight of the composition.

16. A pharmaceutical or cosmetic composition according to claim 12 or 13, in which said composition is for topical administration.

17. A topical pharmaceutical or cosmetic composition according to claim 16, in which said combination of RBR and RBG extracts is present in an amount of 0.1 to 10 wt%, or 0.2 to 5 wt%, or 0.2 to 1 wt%, or of 0.25 wt% or of 0.5 wt% of the total weight of the composition.

1a.

1b.

1c.

1d.

Figure 1

Figure 2

**3a.**

**3b.**

**3c.**

**3d.**

Figure 3

Figure 4

5a.

5b.

**5c.**

**5d.**

Figure 5

Europäisches
Patentamt

European
Patent Office

Office européen
des brevets

# EUROPEAN SEARCH REPORT

**Application Number**

EP 24 38 2624

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/104298 A1 (JOPPE HOLGER [DE] ET AL) 23 April 2009 (2009-04-23)<br><br>* claims 1,7,8 *<br>----- | 1,2,4-6,<br>11,12,<br>14,16 | INV.<br>A61K8/9789<br>A61K36/48<br>A61P17/00<br>A61P17/06 |
| X | US 2011/159122 A1 (FRANK BRUNO [DE]) 30 June 2011 (2011-06-30)<br>* examples 7,8,10,11,13 *<br>----- | 1,2,4,5,<br>12,14,16 | A61P17/10<br>A61Q17/04 |
| Y | Petrova Antoinette: "MODULATION OF ULTRAVIOLET LIGHT-INDUCED SKIN CARCINOGENESIS BY EXTRACTS OF ROOIBOS AND HONEYBUSH USING A MOUSE MODEL: ELUCIDATING POSSIBLE PROTECTIVE MECHANISMS",<br>Biomedical Technology - Masters Degrees,<br>1 January 2009 (2009-01-01), pages 1-179,<br>XP093221463,<br>Retrieved from the Internet:<br>URL:https://etd.cput.ac.za/handle/20.500.11838/1487<br>* pages 49,80,82 *<br>* pages 85-90 *<br>* page 129 *<br>----- | 4-6,8,9,<br>11,12,<br>14,16 | |
| Y | WO 2010/000579 A2 (NESTEC SA [CH]; ROGER OLIVIER YVES [CH]; CRESPY VANESSA [US]) 7 January 2010 (2010-01-07)<br>* claims 1,2,5 *<br>* page 1, lines 1-10 *<br>----- | 4-6,8,9,<br>11,12,<br>14,16 | **TECHNICAL FIELDS SEARCHED (IPC)**<br><br>A61K<br>A61P |
| Y | DATABASE GNPD [Online]<br>MINTEL;<br>20 September 2017 (2017-09-20),<br>anonymous: "Green Rooibos Sun Safety Sun Protection SPF 30",<br>XP093221531,<br>Database accession no. 5106165<br>* page 1 *<br>----- | 4-6,8,9,<br>11,12,<br>14,16 | |

-/--

The present search report has been drawn up for all claims

| Place of search | Date of completion of the search | Examiner |
|---|---|---|
| Munich | 18 November 2024 | Olausson Boulois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 38 2624

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] MINTEL; 11 March 2022 (2022-03-11), anonymous: "Moisturising Day Cream SPF 13", XP093221536, Database accession no. 9443814 * pages 1-2 * | 4-6,8,9, 11,12, 14,16 | |
| Y | PRINGLE NADINE ET AL: "Potential Therapeutic Benefits of Green and Fermented Rooibos (Aspalathus linearis) in Dermal Wound Healing", PLANTA MEDICA, vol. 84, no. 09/10, 1 July 2018 (2018-07-01), pages 645-652, XP093221051, DE ISSN: 0032-0943, DOI: 10.1055/a-0578-8827 Retrieved from the Internet: URL:https://www.thieme-connect.com/products/ejournals/pdf/10.1055/a-0578-8827.pdf> * abstract * | 4-6,8,9, 11,12, 14,16 | |
| A | Nura El-haj ET AL: "Sun protection in a pill: the photoprotective properties of Polypodium leucotomos extract", International Journal of Dermatology, 1 March 2015 (2015-03-01), pages 362-366, XP055532757, England DOI: 10.1111/ijd.12611 Retrieved from the Internet: URL:https://onlinelibrary.wiley.com/doi/10.1111/ijd.12611 * abstract * | 1-17 | TECHNICAL FIELDS SEARCHED (IPC) |

The present search report has been drawn up for all claims

| Place of search | Date of completion of the search | Examiner |
|---|---|---|
| Munich | 18 November 2024 | Olausson Boulois, J |

page 2 of 2

## ANNEX TO THE EUROPEAN SEARCH REPORT
## ON EUROPEAN PATENT APPLICATION NO.

EP 24 38 2624

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2024

| Patent document cited in search report | Publication date | Patent family member(s) | Publication date |
|---|---|---|---|
| US 2009104298 A1 | 23-04-2009 | EP 1959920 A1 | 27-08-2008 |
| | | US 2009104298 A1 | 23-04-2009 |
| | | WO 2007057310 A1 | 24-05-2007 |
| US 2011159122 A1 | 30-06-2011 | EP 2161026 A1 | 10-03-2010 |
| | | EP 2320922 A2 | 18-05-2011 |
| | | JP 5654991 B2 | 14-01-2015 |
| | | JP 2012501990 A | 26-01-2012 |
| | | US 2011159122 A1 | 30-06-2011 |
| | | WO 2010026021 A2 | 11-03-2010 |
| WO 2010000579 A2 | 07-01-2010 | WO 2010000579 A2 | 07-01-2010 |
| | | WO 2010000580 A2 | 07-01-2010 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010000579 A

**Non-patent literature cited in the description**

- **ABDUL NS** ; **MARNEWICK JL**. What has been the focus of Rooibos health research? A bibliometric overview.. *J Herb Med.*, 2023, vol. 37, 100615
- **AKINFENWA AO**. Protective Effects of Linearthin and Other Chalcone Derivatives from Aspalathus linearis (Rooibos) against UVB Induced Oxidative Stress and Toxicity in Human Skin Cells. *Plants Basel Switz.*, 2021, vol. 10 (9), 1936
- **BEDNARSKA K** ; **FECKA I.** Aspalathin and Other Rooibos Flavonoids Trapped α-Dicarbonyls and Inhibited Formation of Advanced Glycation End Products In Vitro. *Int. J. Mol. Sci.*, 2022, vol. 23, 14738
- **SPRINGSTEEN A** ; **YUREK R** ; **FRAZIER M** ; **CARR KF**. In vitro measurement of sun protection factor of sunscreens by diffuse transmittance. *Analytica Chimica Acta.*, 1999, vol. 180 (2-3), 155-164